(19) Europäisches Patentamt
European Patent Office
Office européen des brevets

(11) Veröffentlichungsnummer: **0 302 366 B1**

# (12) EUROPÄISCHE PATENTSCHRIFT

(45) Veröffentlichungstag der Patentschrift: **14.09.94**

(51) Int. Cl.5: **C07D 213/30**, C07D 213/32, C07D 405/04, C07D 213/34, C07D 405/12, C07D 213/57, C07D 213/55, C07D 213/53, A01N 43/40, //C07C49/255

(21) Anmeldenummer: **88112045.5**

(22) Anmeldetag: **26.07.88**

Die Akte enthält technische Angaben, die nach dem Eingang der Anmeldung eingereicht wurden und die nicht in dieser Patentschrift enthalten sind.

## (54) Substituierte Pyridin-Derivate.

(30) Priorität: **05.08.87 DE 3725967**
**07.05.88 DE 3815700**

(43) Veröffentlichungstag der Anmeldung:
**08.02.89 Patentblatt 89/06**

(45) Bekanntmachung des Hinweises auf die Patenterteilung:
**14.09.94 Patentblatt 94/37**

(84) Benannte Vertragsstaaten:
**BE CH DE FR GB IT LI NL SE**

(56) Entgegenhaltungen:
**EP-A- 0 001 399**
**EP-A- 0 111 234**
**EP-A- 0 157 712**
**EP-A- 0 221 844**
**AU-B- 417 269**

(73) Patentinhaber: **BAYER AG**

**D-51368 Leverkusen (DE)**

(72) Erfinder: **Elbe, Hans-Ludwig, Dr.**
**Dasnöckel 59**
**D-5600 Wuppertal 11 (DE)**
Erfinder: **Brandes, Wilhelm, Dr.**
**Eichendorffstrasse 3**
**D-5653 Leichlingen (DE)**
Erfinder: **Dutzmann, Stefan, Dr.**
**Leinenweberweg 33**
**D-4000 Düsseldorf 13 (DE)**
Erfinder: **Hänssler, Gerd, Dr.**
**Am Arenzberg 58a**
**D-5090 Leverkusen 3 (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die Erfindung betrifft neue substituierte Pyridin-Derivate, mehrere Verfahren zu ihrer Herstellung sowie ihre Verwendung als Schädlingsbekämpfungsmittel.

Aus der EP-A 0 001 399 und der EP-A 0 157 712 ist bereits bekannt, daß substituierte Pyridin-Derivate, die zwischen Carbinol-Kohlenstoffatom und Phenoxy- oder Arylthio-Rest eine $CH_2$-Gruppe enthalten, fungizide Eigenschaften besitzen. Entsprechende Stoffe, in denen anstatt der $CH_2$-Gruppe eine $-C(CH_3)_2$-Einheit vorhanden ist, werden aber nicht erwähnt.

Im einzelnen ist bekannt, daß Pyridin-Derivate, wie beispielsweise das 2-(4-Chlor-phenoxy)-1-(2,4-dichlorphenyl)-1-(3-pyridyl)-ethanol oder das 1-(4-Chlor-2-methyl-phenoxy)-3,3-dimethyl -2-(3-pyridyl)-2-butanol oder das 3,3-Dimethyl-1-(2-methyl-phenoxy)-2-(3-pyridyl)-2-butanol oder das 1-(2,4-Dichlorphenoxy)-3,3-dimethyl-2-(3-pyridyl)-2-butanol oder deren Pflanzenverträgliche Säureadditionssalze wie beispielsweise deren Naphthalin-1,5-disulfonate zur Bekämpfung von Pilzen einsetzbar sind (vgl. EP-A 0 001 399).

Die Wirksamkeit dieser vorbekannten Verbindungen ist jedoch insbesondere bei niedrigen Aufwandmengen und Konzentrationen nicht in allen Anwendungsbereichen völlig zufriedenstellend.

Weiterhin werden in der EP-A 0 221 844 fungizid wirksame Pyridin-Derivate beschrieben, die auch einen über $-C(CH_3)_2$-gebundenen Phenoxy-Rest aufweisen können. Verbindungen dieses Typs, in denen keiner der Substituenten am Carbinol-Kohlenstoffatom für Wasserstoff steht, werden aber nicht offenbart.

Es wurden neue substituierte Pyridin-Derivate der allgemeinen Formel (I),

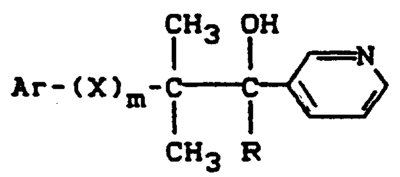

(I)

in welcher

- Ar für gegebenenfalls einfach bis mehrfach, gleich oder verschieden substituiertes Phenyl steht, wobei als Substituenten infrage kommen: Halogen, Cyano, Nitro, jeweils geradkettiges oder verzweigtes Alkyl, Alkoxy, Alkylthio, Alkylsulfinyl oder Alkylsulfonyl mit jeweils 1 bis 8 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen, Cycloalkyl mit 3 bis 6 Kohlenstoffatomen, gegebenenfalls einfach bis mehrfach, gleich oder verschieden durch Halogen substituiertes, zweifach verknüpftes Dioxyalkylen oder jeweils gegebenenfalls einfach bis mehrfach, gleich oder verschieden im Phenylteil durch Halogen substituiertes Phenyl, Phenoxy, Phenylalkyl oder Phenyllkoxy mit jeweils gegebenenfalls 1 bis 4 Kohlenstoffatomen im geradkettigen oder verzweigten Alkyl- oder Alkoxyteil; oder für gegebenenfalls einfach bis mehrfach, gleich oder verschieden substituiertes Naphthyl steht, wobei als Substituenten infrage kommen: Halogen, Cyano oder geradkettiges oder verzweigtes Alkyl mit 1 bis 4 Kohlenstoffatomen;
- X für Sauerstoff, Schwefel, Sulfinyl, Sulfonyl oder für eine der Gruppen $-CH_2-$; $-O-CH_2-$; $-CH_2-O-$; $-O-CH_2-CH_2-$; $-S(O)_n-CH_2-$; $-CH_2-S(O)_n-$ oder $-S(O)_n-CH_2-CH_2-$ steht, wobei
- n jeweils für eine Zahl 0, 1 oder 2 steht,
- R für jeweils geradkettiges oder verzweigtes Alkyl mit 1 bis 6 Kohlenstoffatomen, oder für jeweils gegebenenfalls einfach bis mehrfach, gleich oder verschieden substituiertes Phenoxymethyl oder Phenylthiomethyl steht, wobei als Phenylsubstituenten jeweils die bei dem Rest Ar genannten infrage kommen und
- m für eine Zahl 0 oder 1 steht,

sowie deren pflanzenverträgliche Säureadditionssalze und Metallsalzkomplexe gefunden.

Die Verbindungen der Formel (I) können als geometrische und/oder optische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden, daß man die neuen substituierten Pyridin-Derivate der allgemeinen Formel (I) sowie deren pflanzenverträgliche Säureadditionssalze und Metallsalzkomplexe erhält, wenn man

(a) Ketone der Formel (II),

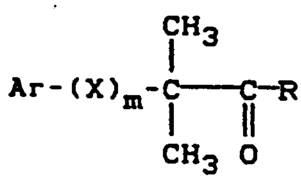

(II)

in welcher

Ar, X, R und m die oben angegebene Bedeutung haben,

mit Pyridyl-Grignard-Verbindungen der Formel (III),

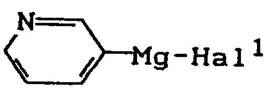

(III)

in welcher

$Hal^1$ für Chlor, Brom oder Iod steht,

gegebenenfalls in Gegenwart eines Verdünnungsmittels umsetzt oder daß man

(b) Pyridylketone der Formel (IV),

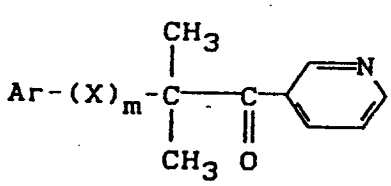

(IV)

in welcher

Ar, X und m die oben angegebene Bedeutung haben,

mit Alkyl-Grignard-Verbindungen der Formel (V),

$R\text{-}Mg\text{-}Hal^2$ (V)

in welcher

R die oben angegebene Bedeutung hat und

$Hal^2$ für Chlor, Brom oder Iod steht,

gegebenenfalls in Gegenwart eines Verdünnungsmittels umsetzt oder daß man alternativ auch, um substituierte Pyridin-Derivate der Formel (Ia),

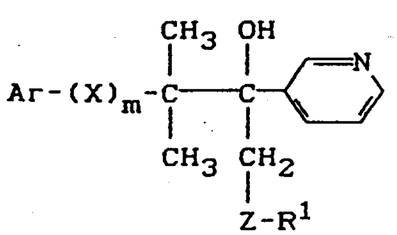

(Ia)

in welcher

Z für Sauerstoff oder Schwefel steht,

$R^1$ für gegebenenfalls einfach bis mehrfach, gleich oder verschieden substituiertes Phenyl steht, wobei als Substituenten die beim Rest Argenannten infrage kommen und

Ar, X und m die oben angegebenen Bedeutungen haben,

zu erhalten,

(c) Pyridyloxirane der Formel (VI),

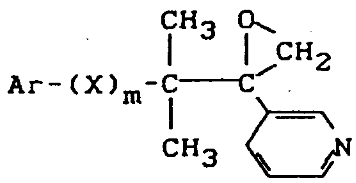

(VI)

in welcher

Ar, X und m die oben angegebene Bedeutung haben,

mit Alkoholen oder Thiolen der Formel (VII),

$R^1$-ZH (VII)

in welcher

$R^1$ und Z die oben angegebene Bedeutung haben,

gegebenenfalls in Gegenwart eines Verdünnungsmittels sowie gegebenenfalls in Gegenwart eines Reaktionshilfsmittel umsetzt und gegebenenfalls anschließend eine Säure oder ein Metallsalz addiert.

Schließlich wurde gefunden, daß die neuen substituierten Pyridin-Derivate der allgemeinen Formel (I) sowie deren Säureadditionssalze und Metallsalzkomplexe eine gute Wirksamkeit gegen Schädlinge insbesondere gegen pilzliche Schädlinge besitzen.

Überraschenderweise zeigen die erfindungsgemäßen substituierten Pyridin-Derivate der allgemeinen Formel (I) eine erheblich bessere fungizide Wirksamkeit als die aus dem Stand der Technik bekannten substituierten Pyridin-Derivate, wie beispielsweise das 2-(4-Chlorphenoxy)-1-(2,4-dichlorphenyl)-1-(3-pyridyl)-ethanol oder das 1-(4-Chlor-3-methyl-phenoxy)-3,3-dimethyl-2-(3-pyridyl)-2-butanol oder das 3,3-Dimethyl-1-(2-methylphenoxy)-2-(3-pyridyl)-2-butanol oder das 1-(2,4-Dichlorphenoxy)-3,3-dimethyl-2-(3-pyridyl)-2-butanol oder deren pflanzenverträgliche Säureadditionssalze wie beispielsweise deren Naphthalin-1,5-disulfonate, welches chemisch und wirkungsmäßig naheliegende Verbindungen sind.

Die erfindungsgemäßen substituierten Pyridin-Derivate sind durch die Formel (I) allgemein definiert. Bevorzugt sind Verbindungen der Formel (I), bei welchen

Ar für gegebenenfalls ein- bis dreifach, gleich oder verschieden substituiertes Phenyl steht, wobei als Substituenten infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Ethylthio, Trifluormethyl, Dichlorfluormethyl, Difluorchlormethyl, Difluorbrommethyl, Trichlormethyl, Fluormethyl, Difluormethyl, Trifluorethyl, Tetrafluorethyl, Trifluorchlorethyl, Pentafluorethyl, Trifluormethoxy, Difluormethoxy, Fluormethoxy, Dichlorfluormethoxy, Difluorchlormethoxy, Difluorbrommethoxy, Trichlormethoxy, Trifluorethoxy, Tetrafluorethoxy, Pentafluorethoxy, Trifluorchlorethoxy, Trifluordichlorethoxy, Difluortrichlorethoxy, Pentachlorethoxy, Trifluormethylthio, Difluormethylthio, Fluormethylthio, Difluorchlormethylthio, Dichlorfluormethylthio, Difluorethylthio, Difluorbrommethylthio, Trichlormethylthio, Trifluorethylthio, Tetrafluorethylthio, Pentafluorethylthio, Trifluorchlorethylthio, Trifluordichlorethylthio, Pentachlorethylthio, Methylsulfinyl, Trifluormethylsulfinyl, Dichlorfluormethylsulfinyl, Difluorchlormethylsulfinyl, Fluormethylsulfinyl, Difluormethylsulfinyl, Methylsulfonyl, Trifluormethylsulfonyl, Dichlorfluormethylsulfonyl, Difluorchlormethylsulfonyl, Fluormethylsulfonyl, Difluormethylsulfonyl, Methoxycarbonyl, Ethoxycarbonyl, Methoximinomethyl, Ethoximinomethyl, Methoximinoethyl, Ethoximinoethyl, Difluordioxymethylen, Tetrafluordioxyethylen, Trifluordioxyethylen, Difluordioxyethylen, Dioxymethylen, Dioxyethylen sowie jeweils gegebenenfalls ein- bis dreifach durch Fluor oder Chlor substituiertes Phenyl oder Phenoxy;

oder für gegebenenfalls ein- bis dreifach, gleich oder verschieden substituiertes $\alpha$-Naphthyl oder $\beta$-Naphthyl steht, wobei als Substituenten jeweils infrage kommen: Fluor, Chlor, Brom, Cyano, Methyl, Ethyl, n- oder i-Propyl;

X für Sauerstoff, Schwefel, Sulfinyl, Sulfonyl oder für eine der Gruppen $-CH_2-$; $-O-CH_2-$; $-CH_2-O-$; $-O-CH_2-CH_2-$; $-S(O)_n-CH_2-$; $-CH_2-S(O)_n-$ oder $-S(O)_n-CH_2-CH_2-$ steht, wobei

n jeweils für eine Zahl 0, 1 oder 2 steht,

R für Methyl, Ethyl, n- oder i-Propyl, n-, i-, s- oder t-Butyl oder für jeweils gegebenenfalls ein- bis dreifach, gleich oder verschieden substituiertes Phenoxymethyl oder Phenylthiomethyl steht, wobei als Phenylsubstituenten jeweils die bei dem Rest Ar genannten infrage kommen und

m für eine Zahl 0 oder 1 steht.

Bevorzugte erfindungsgemäß verwendbare Verbindungen sind auch Additionsprodukte aus Säuren und denjenigen substituierten Pyridin-Derivaten der Formel (I), in denen die Substituenten Ar, X, m und R die Bedeutung haben, die bereits vorzugsweise für diese Substituenten genannt wurden.

Zu den Säuren, die addiert werden können, gehören vorzugsweise Halogenwasserstoffsäuren, wie z.B. Chlorwasserstoffsäure und Bromwasserstoffsäure, insbesondere Chlorwasserstoffsäure, ferner Phosphorsäure, Salpetersäure, Schwefelsäure, mono-, bi- und trifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure, Sulfonsäuren, wie p-Toluolsulfonsäure oder 1,5-Naphthalindisulfonsäure sowie Saccharin oder Thiosaccharin.

Außerdem bevorzugte erfindungsgemäß verwendbare Verbindungen sind Additionsprodukte aus Salzen von Metallen der II. bis IV. Haupt- und der I. und II. sowie IV. bis VIII. Nebengruppe und denjenigen substituierten Pyridin-Derivaten der Formel (I), in denen die Substituenten Ar, X, m und R die Bedeutungen haben, die bereits vorzugsweise für diese Substituenten genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu pflanzenverträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Im einzelnen seien außer den bei den Herstellungsbeispielen genannten Verbindungen die folgenden substituierten Pyridin-Derivate der allgemeinen Formel (I) genannt:

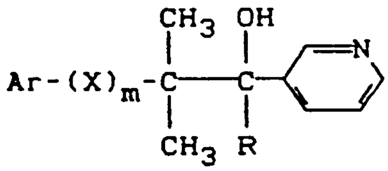 (I)

| Ar | $-(X)_m-$ | R |
|---|---|---|
| 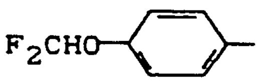 | $-CH_2-$ | $-CH_3$ |
| 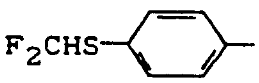 | $-CH_2-$ | $-CH_3$ |
| 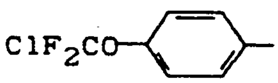 | $-CH_2-$ | $-CH_3$ |
| 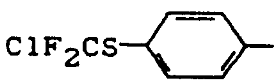 | $-CH_2-$ | $-CH_3$ |
| 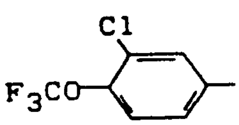 | $-CH_2-$ | $-CH_3$ |
| 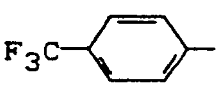 | $-CH_2-$ | $-CH_3$ |
| 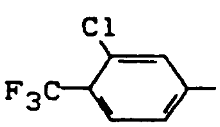 | $-CH_2-$ | $-CH_3$ |

| Ar | $-(X)_m-$ | R |
|---|---|---|
| 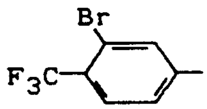 | $-CH_2-$ | $-CH_3$ |
| 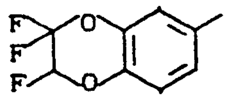 | $-CH_2-$ | $-CH_3$ |
| 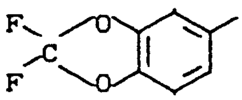 | $-CH_2-$ | $-CH_3$ |
| 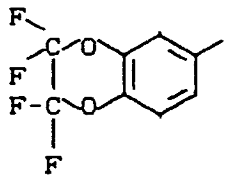 | $-CH_2-$ | $-CH_3$ |
| 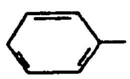 | - | $-CH_3$ |
| 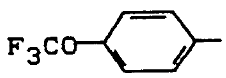 | - | $-CH_3$ |
| 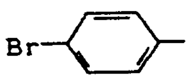 | - | $-CH_3$ |
| 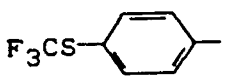 | - | $-CH_3$ |
| 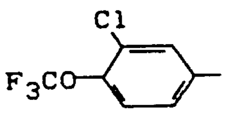 | - | $-CH_3$ |
| 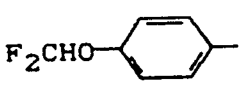 | - | $-CH_3$ |

| Ar | -(X)$_m$- | R |
|---|---|---|
| 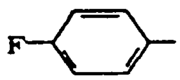 | - | $-CH_3$ |
| 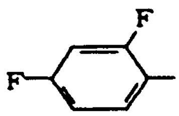 | - | $-CH_3$ |
| 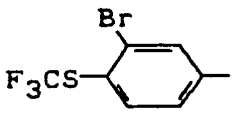 | - | $-CH_3$ |
| 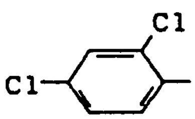 | - | $-CH_3$ |
| 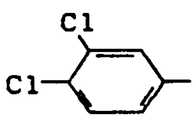 | - | $-CH_3$ |
| 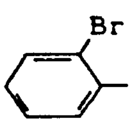 | - | $-CH_3$ |
| 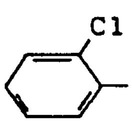 | - | $-CH_3$ |
| 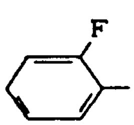 | - | $-CH_3$ |
| 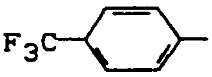 | - | $-CH_3$ |

| Ar | $-(X)_m-$ | R |
|---|---|---|
| 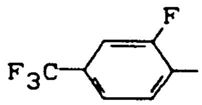 | - | $-CH_3$ |
| 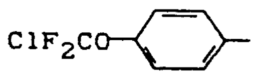 | - | $-CH_3$ |
| 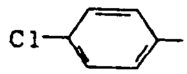 | $-O-CH_2-CH_2-$ | 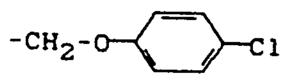 |
| 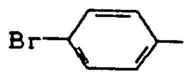 | $-O-CH_2-CH_2-$ | 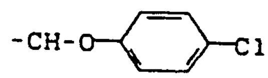 |
| 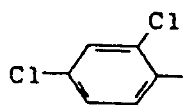 | $-O-CH_2-CH_2-$ | 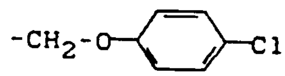 |
| 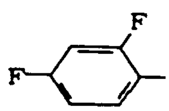 | $-O-CH_2-CH_2-$ | 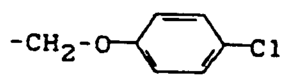 |
| 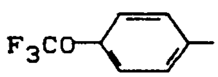 | $-O-CH_2-CH_2-$ | 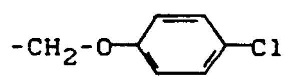 |
| 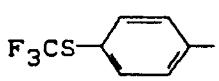 | $-O-CH_2-CH_2-$ | 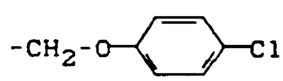 |
| 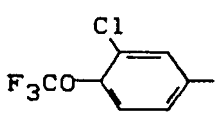 | $-O-CH_2-CH_2-$ | 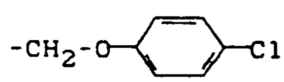 |
| 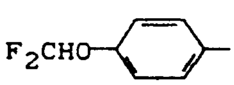 | $-O-CH_2-CH_2-$ | 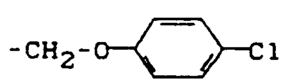 |

| Ar | $-(X)_m-$ | R |
|---|---|---|
| 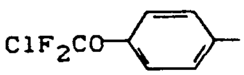 | $-O-CH_2-CH_2-$ | 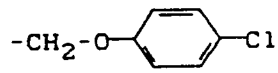 |
| 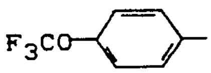 | $-O-CH_2-$ | $-CH_3$ |
| 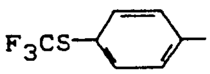 | $-O-CH_3-$ | $-CH_3$ |
| 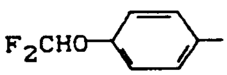 | $-O-CH_2-$ | $-CH_3$ |
| 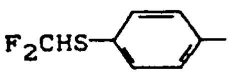 | $-O-CH_2-$ | $-CH_3$ |
| 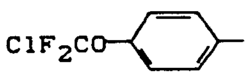 | $-O-CH_2-$ | $-CH_3$ |
| 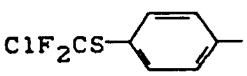 | $-O-CH_2-$ | $-CH_3$ |
| 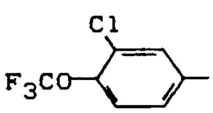 | $-O-CH_2-$ | $-CH_3$ |
| 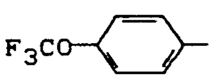 | O | 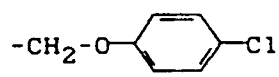 |
| 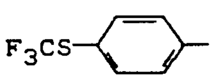 | O | 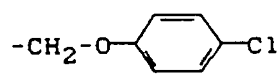 |
| 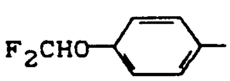 | O | 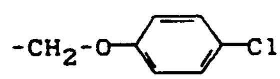 |

| Ar | $-(X)_m-$ | R |
|---|---|---|
| 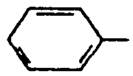 | O | 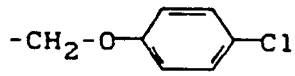 |
| 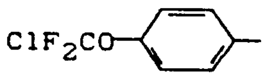 | O | 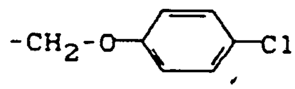 |
| 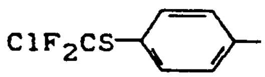 | O | 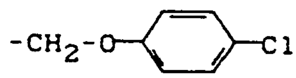 |
| 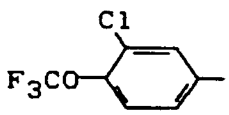 | O | 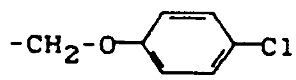 |
| 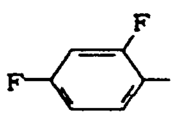 | O | 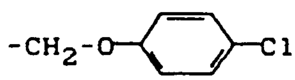 |
| 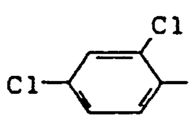 | O | 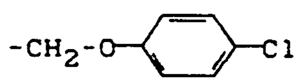 |
| 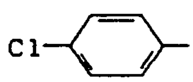 | O | 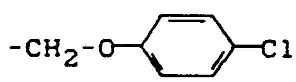 |
| 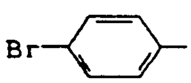 | O | 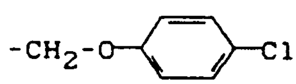 |
| 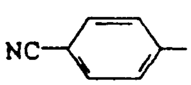 | O | 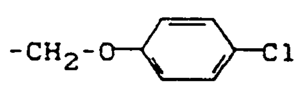 |
| 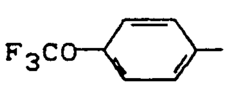 | O | 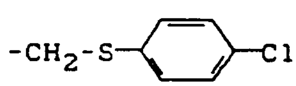 |

| Ar | $-(X)_m-$ | R |
|---|---|---|
| 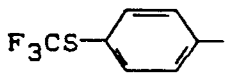 | O | 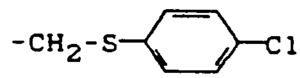 |
| 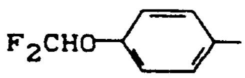 | O | 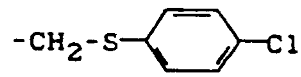 |
| 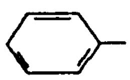 | O | 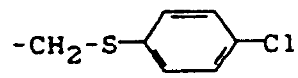 |
| 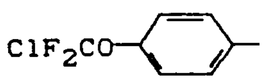 | O | 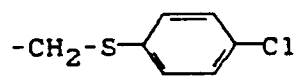 |
| 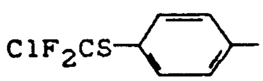 | O | 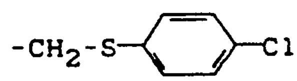 |
| 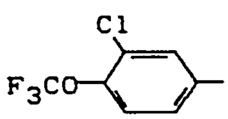 | O | 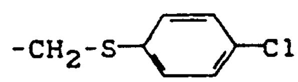 |
| 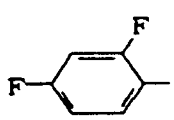 | O | 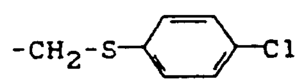 |
| 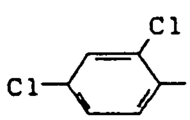 | O | 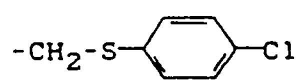 |
| 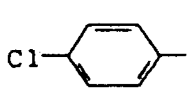 | O | 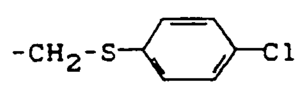 |
| 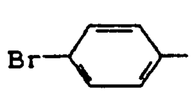 | O | 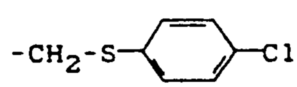 |

| Ar | $-(X)_m-$ | R |
|---|---|---|
| 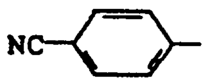 | O | 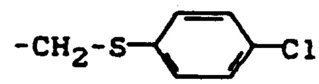 |

Verwendet man beispielsweise 3-(4-Chlorphenoxy)-3-methyl-butan-2-on und 3-Pyridylmagnesiumbromid als Ausgangstoffe, so läßt sich der Reaktionsablauf des erfindungsgemäßen Verfahrens (a) durch das folgende Formelschema darstellen:

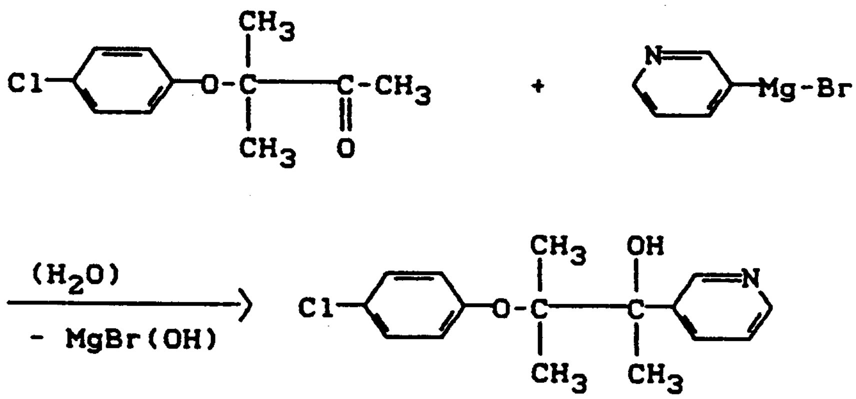


Verwendet man beispielsweise 3-(4-Fluorphenyl)-2,2-dimethyl-1-(3-pyridyl)-propan-1-on und Allylmagnesiumbromid als Ausgangsstoffe, so läßt sich der Reaktionsablauf des erfindungsgemäßen Verfahrens (b) durch das folgende Formelschema darstellen:

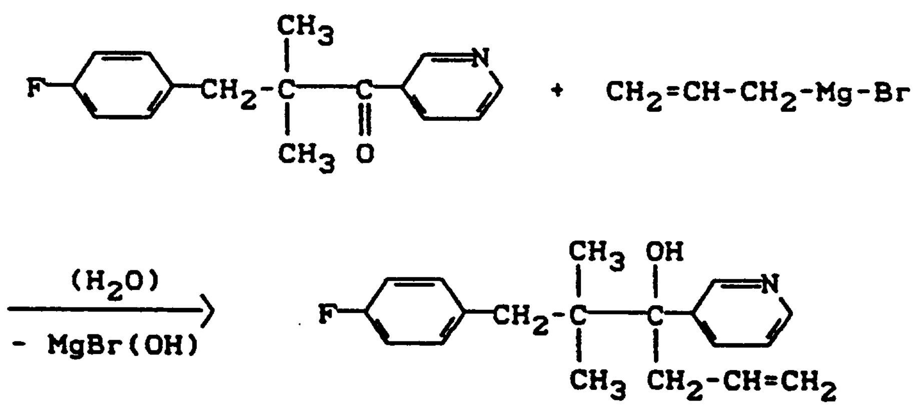


Verwendet man beispielsweise 2-[2-(2,4-Dichlorphenoxy)-2-propyl]-2-(3-pyridyl)-oxiran und 4-Chlorphenol als Ausgangsstoffe, so läßt sich der Reaktionsablauf des erfindungsgemäßen Verfahrens (c) durch das folgende Formelschema darstellen:

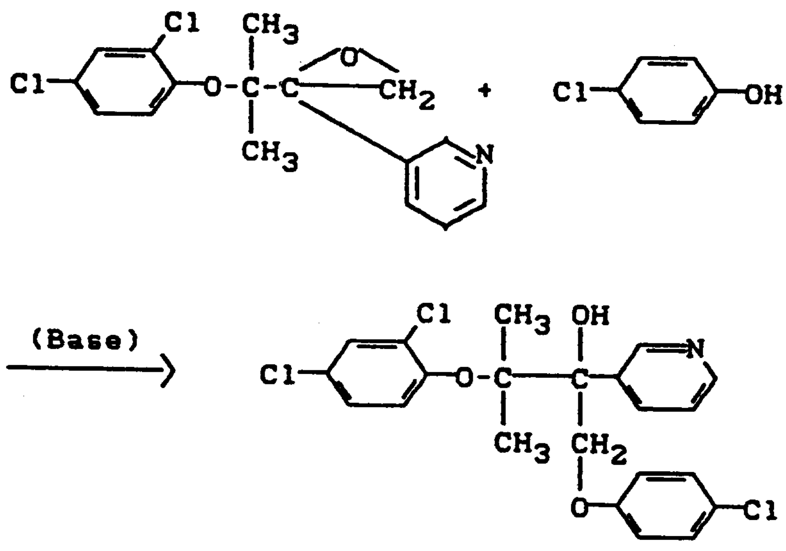


Die zur Durchführung des erfindungsgemäßen Verfahrens (a) als Ausgangsstoffe benötigten Ketone sind durch die Formel (II) allgemein definiert. In dieser Formel (II) stehen R, Ar, X und m vorzugsweise für diejenigen Reste und Indices, die bereits im Zusammenhang mit der Beschreibung der erfindungsgemäßen Stoffe der Formel (I) als bevorzugt für diese Substituenten und Indices genannt wurden.

Die Ketone der Formel (II) sind bekannt oder können in Analogie zu bekannten Verfahren erhalten werden (vgl. z.B. DE-A 3 210 725 oder DE-A 3 048 266).

Die zur Durchführung des erfindungsgemäßen Verfahrens (a) weiterhin als Ausgangsstoffe benötigten Pyridyl-Grignard-Verbindungen sind durch die Formel (III) allgemein definiert. In dieser Formel (III) steht $Hal^1$ für Chlor, Brom oder Iod. Die Pyridyl-Grignard-Verbindungen der Formel (III) sind ebenfalls bekannt (vgl. z.B. EP-A 221 844 oder Angew. Chem. Int. Ed. Engl. 8, 279 [1969]).

Die zur Durchführung des erfindungsgemäßen Verfahrens (b) als Ausgangsstoffe benötigten Pyridylketone sind durch die Formel (IV) allgemein definiert. In dieser Formel (IV) stehen Ar, X und m vorzugsweise für diejenigen Reste und Indices, die bereits im Zusammenhang mit der Beschreibung der erfindungsgsmäßen Stoffe der Formel (I) als bevorzugt für diese Substituenten und Indices genannt wurden.

Die Pyridylketone der Formel (IV) sind teilweise bekannt (vgl. z.B. EP-A 221 844) oder Bestandteil einer eigenen nicht vorveröffentlichten Anmeldung und erhältlich in Analogie zu bekannten Verfahren, beispielsweise wenn man Ester der Formel (VIII),

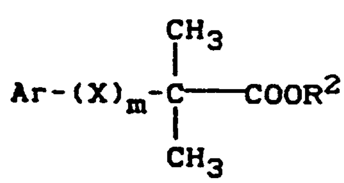 (VIII)

in welcher

$R^2$ für Methyl oder Ethyl steht,

mit Pyridyl-Grignard-Verbindungen der Formel (III),

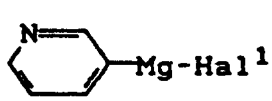 (III)

in welcher

$Hal^1$ für Chlor, Brom oder Iod steht,

gegebenenfalls in Gegenwart eines Verdünnungsmittels, wie beispielsweise Diethylether oder Tetrahydrofuran, bei Temperaturen zwischen - 20 °C und + 60 °C umsetzt; oder wenn man Carbinole der Formel (IX),

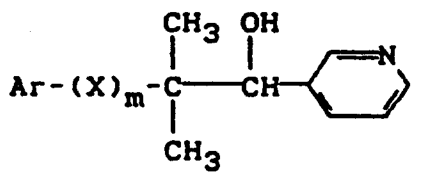

(IX)

in welcher

Ar, X und m die oben angegebene Bedeutung haben,

mit einem üblichen Oxidationsmittel wie beispielweise Chromtrioxid in Eisessig oxidiert (vgl. hierzu auch beispielsweise Tetrahedron Letters 1978, 1651 - 1660).

Die Ester der Formel (VIII) sind bekannt oder erhältlich in Analogie zu bekannten Verfahren (vgl. z.B. EP-A 221 844 oder Przem. Chem. 59, 495 - 498 [1980] bzw. CA 94: 102 975 e).

Die Carbinole der Formel (IX) sind teilweise bekannt (vgl. EP-A 221 844), teilweise sind sie Gegenstand einer eigenen, nicht vorveröffentlichten Patentanmeldung und erhältlich in Analogie zu bekannten Verfahren.

Die zur Durchführung des erfindungsgemäßen Verfahrens (b) weiterhin als Ausgangsstoffe benötigten Alkyl-Grignard-Verbindungen sind durch die Formel (V) allgemein definiert. In dieser Formel (V) steht R vorzugsweise für diejenigen Reste, die bereits im Zusammenhang mit der Beschreibung der erfindungsgemäßen Stoffe der Formel (I) als bevorzugt für diesen Substituenten genannt wurden.

$Hal^2$ steht für Chlor, Brom oder Iod.

Die Alkyl-Grignard-Verbindungen der Formel (V) sind allgemein bekannte Verbindungen der organischen Chemie.

Die zur Durchführung des erfindungsgemäßen Verfahrens (c) als Ausgangsstoffe benötigten Pyridyloxirane sind durch die Formel (VI) allgemein definiert. In dieser Formel (VI) stehen Ar, X und m vorzugsweise für diejenigen Reste und Indices, die bereits im Zusammenhang mit der Beschreibung der erfindungsgemäßen Stoffe der Formel (I) als bevorzugt für diese Substituenten und Indices genannt wurden.

Die Pyridyloxirane der Formel (VI) sind noch nicht bekannt.

Man erhält sie, wenn man Pyridylketone der Formel (IV),

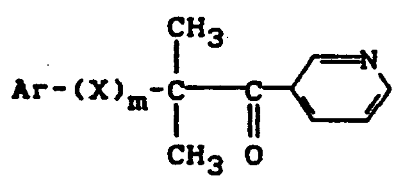

(IV)

in welcher

Ar, X und m die oben angegebene Bedeutung haben,

entweder mit Dimethyloxosulfonium-methylid der Formel (X)

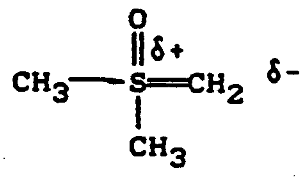

(X)

in bekannter Weise in Gegenwart eines Verdünnungsmittels wie beispielsweise Dimethylsulfoxid bei Temperaturen zwischen 20 °C und 80 °C umsetzt (vgl. z.B. J. Amer. Chem. Soc. 87, 1363 - 1364 [1965]), oder mit Trimethylsulfoniummethylsulfat der Formel (XI),

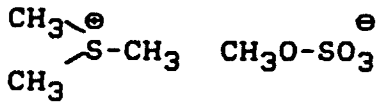 (XI)

in bekannter Weise in Gegenwart eines Verdünnungsmittels wie beispielsweise Acetonitril und in Gegenwart einer Base wie beispielsweise Natriummethylat bei Temperaturen zwischen 0 °C und 60 °C umsetzt (vgl. z.B. Heterocycles 8, 397 [1977]).

Die so erhältlichen Pyridyloxirane der Formel (VI) können gegebenenfalls ohne Isolierung direkt aus dem Reaktionsgemisch heraus weiter umgesetzt werden.

Die zur Durchführung des erfindungsgemäßen Verfahrens (c) weiterhin als Ausgangsstoffe benötigten Alkohole oder Thiole sind durch die Formel (VII) allgemein definiert. In dieser Formel (VII) steht $R^1$ für gegebenenfalls ein- bis dreifach, gleich oder verschieden substituiertes Phenyl, wobei als Substituenten infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Ethylthio, Trifluormethyl, Dichlorfluormethyl, Difluorchlormethyl, Difluorbrommethyl, Trichlormethyl, Fluormethyl, Difluormethyl, Trifluorethyl, Tetrafluorethyl, Trifluorchlorethyl, Pentafluorethyl, Trifluormethoxy, Difluormethoxy, Fluormethoxy, Dichlorfluormethoxy, Difluorchlormethoxy, Difluorbrommethoxy, Trichlormethoxy, Trifluorethoxy, Tetrafluorethoxy, Pentafluorethoxy, Trifluorchlorethoxy, Trifluordichlorethoxy, Difluortrichlorethoxy, Pentachlorethoxy, Trifluormethylthio, Difluormethylthio, Fluormethylthio, Difluorchlormethylthio, Dichlorfluormethylthio, Difluorethylthio, Difluorbrommethylthio, Trichlormethylthio, Trifluorethylthio, Tetrafluorethylthio, Pentafluorethylthio, Trifluorchlorethylthio, Trifluordichlorethylthio, Pentachlorethylthio, Methylsulfinyl, Trifluormethylsulfinyl, Dichlorfluormethylsulfinyl, Difluorchlormethylsulfinyl, Fluormethylsulfinyl, Difluormethylsulfinyl, Methylsulfonyl, Trifluormethylsulfonyl, Dichlorfluormethylsulfonyl, Difluorchlormethylsulfonyl, Fluormethylsulfonyl, Difluormethylsulfonyl, Methoxycarbonyl, Ethoxycarbonyl, Methoximinomethyl, Ethoximinomethyl, Methoximinoethyl, Ethoximinoethyl, Difluordioxymethylen, Tetrafluordioxyethylen, Trifluordioxyethylen, Difluordioxyethylen, Dioxymethylen, Dioxyethylen sowie jeweils gegebenenfalls einbis dreifach durch Fluor oder Chlor substituiertes Phenyl oder Phenoxy.

Z steht für Sauerstoff oder Schwefel.

Die Alkohole oder Thiole der Formel (VII) sind allgemein bekannte Verbindungen der organischen Chemie.

Als Verdünnungsmittel zur Durchführung des erfindungsgemäßen Verfahrens (a) kommen inerte organische Lösungsmittel infrage. Hierzu gehören insbesondere aliphatische, alicyclische oder aromatische Kohlenwasserstoffe, wie beispielsweise Benzin, Benzol, Toluol, Xylol, Petrolether, Hexan oder Cyclohexan, Ether, wie Diethylether, Diisopropylether, Dioxan, Tetrahydrofuran oder Ethylenglykoldimethyl- oder -diethylether oder Amide, wie Hexamethylphosphorsäuretriamid.

Die Reaktionstemperaturen können bei der Durchführung des erfindungsgemäßen Verfahrens (a) in einem größeren Bereich variiert werden. Im allgemeinen arbeitet man bei Temperaturen zwischen - 50 °C und + 150 °C, vorzugsweise bei Temperaturen zwischen - 20 °C und + 120 °C.

Zur Durchführung des erfindungsgemäßen Verfahrens (a) setzt man pro Mol an Keton der Formel (II) im allgemeinen 1.0 bis 3.0 Mol, vorzugsweise 1.0 bis 1.5 Mol an Pyridyl-Grignard-Verbindung der Formel (III) ein. Die Reaktionsdurchführung erfolgt nach allgemein üblichen Methoden.
Dabei kann die Umsetzung erforderlichenfalls in Gegenwart eines geeigneten Inertgases, wie beispielsweise Stickstoff oder Helium durchgeführt werden. Es ist auch möglich, die als Reaktionspartner eingesetzte Pyridyl-Grignard-Verbindung der Formel (III) aus geeigneten Ausgangsverbindungen wie beispielsweise 3-Brompyridin und Isopropylmagnesiumbromid in einer vorgelagerten Reaktion direkt im Reaktionsgefäß herzustellen und ohne Isolierung im Eintopfverfahren mit den Ketonen der Formel (II) weiter umzusetzen.
Die Aufarbeitung und Isolierung der Reaktionsprodukte erfolgt ebenfalls nach üblichen Methoden (vgl. die Herstellungsbeispiele).

Als Verdünnungsmittel zur Durchführung des erfindungsgemäßen Verfahrens (b) kommen ebenfalls inerte organische Lösungsmittel infrage. Vorzugsweise verwendet man die bei Verfahren (a) genannten organischen Lösungsmittel als Verdünnungsmittel.

Die Reaktionstemperaturen können bei der Durchführung des erfindungsgemäßen Verfahrens (b) in einem größeren Bereich variiert werden. Im allgemeinen arbeitet man bei Temperaturen zwischen - 50 °C und + 150 °C, vorzugsweise bei Temperaturen zwischen - 20 °C und + 120 °C.

Zur Durchführung des erfindungsgemäßen Verfahrens (b) setzt man pro Mol an Pyridylketon der Formel (IV) im allgemeinen 1.0 bis 3.0 Mol, vorzugsweise 1.0 bis 1.5 Mol an Alkyl-Grignard-Verbindung der

Formel (V) ein. Die Reaktionsdurchführung, Aufarbeitung und Isolierung der Reaktionsprodukte erfolgt nach allgemein üblichen Methoden.

Als Verdünnungsmittel zur Durchführung des erfindungsgemäßen Verfahrens (c) kommen inerte organische Lösungsmittel infrage. Hierzu gehören insbesondere aliphatische, alicyclische oder aromatische Kohlenwasserstoffe, wie beispielsweise Benzin, Benzol, Toluol, Xylol, Petrolether, Hexan oder Cyclohexan, Ether, wie Diethylether, Dioxan, Tetrahydrofuran oder Ethylenglykoldimethyl- oder -diethylether, Ketone, wie Aceton oder Butanon, Nitrile, wie Acetonitril oder Propionitril oder Amide, wie Dimethylformamid, Dimethylacetamid, N-Methylformanilid, N-Methylpyrrolidon oder Hexamethylphosphorsäuretriamid.

Es ist gegebenenfalls auch möglich, die als Reaktionspartner verwendeten Alkohole oder Thiole der Formel (VII) in entsprechendem Überschuß gleichzeitig als Verdünnungsmittel einzusetzen.

Das erfindungsgemäße Verfahren (c) wird vorzugsweise in Gegenwart eines geeigneten basischen Reaktionshilfsmittels durchgeführt. Als solche kommen alle üblicherweise verwendbaren anorganischen und organischen Basen in Frage. Vorzugsweise verwendet man Alkalimetallhydride, -hydroxide, -amide, -carbonate oder -hydrogencarbonate, wie beispielsweise Natriumhydrid, Natriumamid, Natriumhydroxid, Natriumcarbonat oder Natriumhydrogencarbonat oder auch tertiäre Amine, wie beispielsweise Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (c) in einem größeren Bereich variiert werden. Im allgemeinen arbeitet man bei Temperaturen zwischen 0 °C und 200 °C, vorzugsweise bei Temperaturen zwischen 60 °C und 150 °C.

Zur Durchführung des erfindungsgemäßen Verfahrens (c) setzt man pro Mol an Pyridyloxiran der Formel (VI) im allgemeinen 1.0 bis 20.0 Mol, vorzugsweise 1.0 bis 5.0 Mol an Alkohol oder Thiol der Formel (VII) und gegebenenfalls 1.0 bis 5.0 Mol, vorzugsweise 1.0 bis 2.0 Mol an basischem Reaktionshilfsmittel ein.

Die Reaktionsdurchführung, Aufarbeitung und Isolierung der Reaktionsprodukte erfolgt nach allgemein üblichen Methoden (vgl. z.B. DE-A 3 427 844).

Gegebenenfalls kann man die mit Hilfe der erfindungsgemäßen Verfahren (a), (b) oder (c) erhältlichen substituierten Pyridin-Derivate der Formel (I) nach üblichen Methoden derivatisieren, beispielsweise indem man die Hydroxygruppe mit üblichen Alkylierungs- oder Acylierungsmitteln umsetzt. Die so erhältlichen Alkylierungs- oder Acylierungsprodukte der erfindungsgemäßen Verbindungen der Formel (I) besitzen ebenfalls gute fungizide Wirksamkeit.

Zur Herstellung von Pflanzenverträglichen Säureadditionssalzen der Verbindungen der Formel (I) kommen vorzugsweise folgende Säuren infrage: Halogenwasserstoffsäuren, wie z.B. Chlorwasserstoffsäure und Bromwasserstoffsäure, insbesondere Chlorwasserstoffsäure, ferner Phosphorsäure, Salpetersäure, Schwefelsäure, mono-, bi- und trifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure, Sulfonsäuren, wie z.B. p-Toluolsulfonsäure und 1,5-Naphthalindisulfonsäure sowie Saccharin oder Thiosaccharin.

Die Säureadditionssalze der Verbindungen der Formel (I) können in einfacher Weise nach üblichen Salzbildungsmethoden, wie z.B. durch Lösen einer Verbindung der Formel (I) in einem geeigneten inerten Lösungsmittel und Hinzufügen der Säure, wie z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise Salze von Metallen der II. bis IV. Haupt- und der I. und II. sowie IV. bis VIII. Nebengruppe infrage, wobei Kupfer, Zink, Mangan, Magnesium, Zinn, Eisen und Nickel beispielhaft genannt seien.

Als Anionen von Salzen kommen solche in Betracht, die sich vorzugsweise von folgenden Säuren ableiten: Halogenwasserstoffsäure, wie z.B. Chlorwasserstoffsäure und Bromwasserstoffsäure, ferner Phosphorsäure, Salpetersäure und Schwefelsäure.

Die Metallsalzkomplexe von Verbindungen der Formel (I) können in einfacher Weise nach üblichen Verfahren erhalten werden, so z.B. durch Lösen des Metallsalzes in Alkohol, z.B. Ethanol und Hinzufügen zur Verbindung der Formel (I). Man kann Metallsalz-Komplexe in bekannter Weise, z.B. durch Abfiltrieren isolieren und gegebenenfalls durch Umkristallisation reinigen.

Die erfindungsgemäßen Wirkstoffe weisen eine starke Wirkung gegen Schädlinge auf und können zur Bekämpfung von unerwünschten Schadorganismen praktisch eingesetzt werden. Die Wirkstoffe können für den Gebrauch als Pflanzenschutzmittel insbesondere als Fungizide singesetzt werden.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Getreidekrankheiten, wie beispielsweise gegen den Erreger des echten Getreidemehltaus (Erysiphe graminis), gegen den Erreger der Braunfleckigkeit des Weizens (Leptosphaeria nodorum), gegen den Erreger der Braunfleckenkrankheit der Gerste (Cochliobolus sativus) oder gegen den Erreger der Blattfleckenkrankheit der Gerste (Pyrenophora teres), zur Bekämpfung von Reiskrankheiten, wie beispielsweise zur Bekämpfung der Reisfleckenkrankheit (Pyricularia oryzae) oder zur Bekämpfung der Reisstengelkrankheit (Pellicularia sasakii) oder zur Bekämpfung von Krankheiten im Obst und Gemüsebau, wie beispielsweise gegen den Erreger des Bohnengrauschimmels (Botrytis cinerea) oder gegen den Erreger des Apfelschorfes (Venturia inaequalis) sowie gegen echte Mehltau- und Rostpilze in Gemüsekulturen einsetzen. Darüberhinaus besitzen die erfindungsgemäßen Wirkstoffe eine besonders breite fungizide Wirksamkeit bei invitro-Versuchen.

In entsprechenden Aufwandmengen besitzen die erfindungsgemäßen Wirkstoffe darüberhinaus auch eine wachstumsregulierende Wirkung bei Kulturpflanzen.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und anderen Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

Herstellungsbeispiele

Beispiel 1

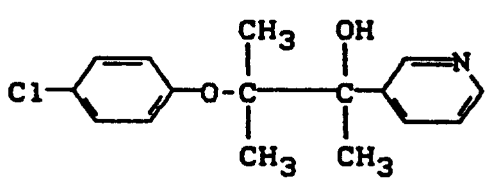

(Verfahren a)

Zu 28,4 g (0,18 Mol) 3-Brompyridin in 95 ml Diethylether gibt man bei Raumtemperatur unter Rühren 26,5 g (0,18 Mol) Isopropylmagnesiumbromid in 130 ml Diethylether, rührt 30 Minuten und gibt dann tropfenweise unter Rühren 31,9 g (0,15 Mol) 3-Methyl-3-(4-chlorphenoxy)-butan-2-on (vgl. z.B. EP-A 54 865)

zu, rührt nach beendeter Zugabe 4 Stunden bei Rückflußtemperatur, kühlt ab auf 25 °C und tropft unter Eiskühlung 50 ml Wasser zu. Die Mischung wird mit verdünnter Salzsäure auf pH 5 bis pH 6 gebracht, die organische Phase abgetrennt, mit Wasser gewaschen, über Natriumsulfat getrocknet und im Vakuum eingeengt. Der Rückstand wird durch Chromatographie an Kieselgel (Essigester / Cyclohexan 3 : 1) gereinigt.

Man erhält 12,7 g (29 % der Theorie) an 3-(4-Chlorphenoxy)-3-methyl-2-(3-pyridyl)-butan-2-ol vom Schmelzpunkt 140 °C - 141 °C.

Beispiel 2

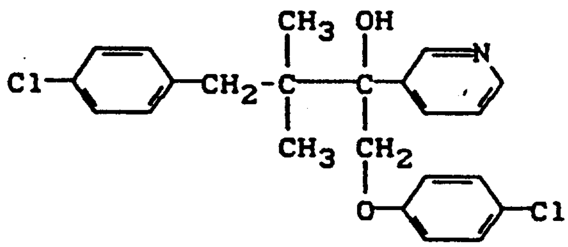

(Verfahren a)

Zu 17,6 g (0,112 Mol) 3-Brompyridin in 90 ml Tetrahydrofuran gibt man bei Raumtemperatur tropfenweise 16,5 g (0,112 Mol) Isopropylmagnesiumbromid in 90 ml Diethylether und rührt nach beendeter Zugabe 30 Minuten nach. Darauf gibt man tropfenweise unter Rühren 31,4 g (0,093 Mol) 1-(4-Chlorphenoxy)-4-(4-chlorphenyl)-3,3-dimethylbutan-2-on in 70 ml Diethylether zu, rührt nach beendeter Zugabe 4 Stunden bei Rückflußtemperatur, kühlt auf Raumtemperatur ab, gibt unter Kühlung 50 ml Wasser zu, stellt mit verdünnter Salzsäure auf pH 6 - pH 7 ein, trennt die organische Phase ab, wäscht mit Wasser, trocknet über Natriumsulfat und engt im Vakuum ein. Der Rückstand wird durch Chromatographie an Kieselgel (Essigster / Cyclohexan 1 : 3 ) gereinigt.

Man erhält 10,8 g (28 % der Theorie) an 1-(4-Chlorphenoxy)-4-(4-chlorphenyl)-3,3-dimethyl-2-(3-pyridyl)butan-2-ol vom Schmelzpunkt 46 °C - 47 °C.

Herstellung der Ausgangsverbindung

Beispiel II-1

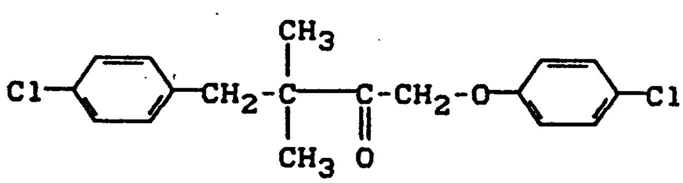

46,3 g (0,36 Mol) 4-Chlorphenol und 49,7 g (0,36 Mol) Kaliumcarbonat in 150 ml Toluol werden 2 Stunden unter Rückfluß über einem Wasserabscheider erhitzt. Danach gibt man bei 60 °C tropfenweise unter Rühren 87 g (0,3 Mol) 1-Brom-4-(4-chlorphenyl)-3,3-dimethyl-butan-2-on (vgl. z.B. DE-A 3 229 273) in 150 ml Toluol zu, rührt nach beendeter Zugabe weitere 5 Stunden bei 100 °C, kühlt ab, saugt ausgefallenen Niederschlag ab, wäscht das Filtrat mit verdünnter Natronlauge und Wasser, trocknet über Natriumsulfat und entfernt das Lösungsmittel im Vakuum.

Man erhält 80,7 g (80 % der Theorie) an 1-(4-Chlorphenoxy-4-(4-chlorphenyl)-3,3-dimethylbutan-2-on als Öl, welches ohne weitere Reinigung in die folgende Reaktion eingesetzt wird.

In entsprechender Weise und gemäß den allgemeinen Angaben zur Herstellung erhält man die folgenden substituierten Pyridin-Derivate der allgemeinen Formel (I):

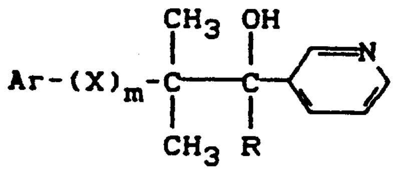 , (I)

| Bsp. Nr. | Ar | $-(X)_m-$ | R | physikalische Eigenschaften |
|---|---|---|---|---|
| 3 | 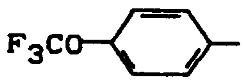 | $-CH_2-$ | $-CH_3$ | $^1H$-NMR*): 1,73(s); 0,83(s); 0,77(s) |
| 4 | 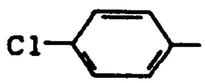 | $-CH_2-$ | $-CH_3$ | $^1H$-NMR*): 1,70(s); 0,83(s); 0,77(s) |
| 5 | 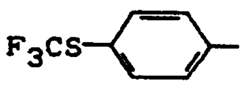 | $-CH_2-$ | $-CH_3$ | $n_D^{20}$ 1.5251 |
| 6 | 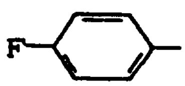 | -O- | $-CH_3$ | Fp. 125 °C |
| 7 | 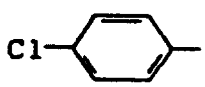 | $-O-CH_2-$ | $-CH_3$ | $n_D^{20}$ 1.5471 |

| Bsp. Nr. | Ar | $-(X)_m-$ | R | physikalische Eigenschaften |
|---|---|---|---|---|
| 8 | 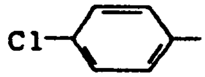 | -S- | $-CH_3$ | Fp. 116 °C |
| 9 | 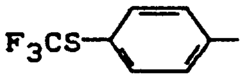 | $-CH_2-$ | 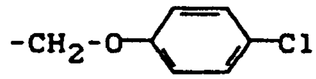 | $n_D^{20}$ 1.5704 |
| 10 | 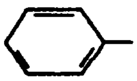 | $-CH_2-$ | 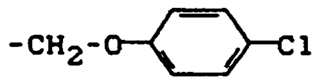 | Fp. 40-42° C |
| 11 | 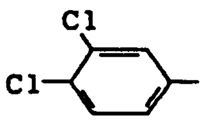 | $-CH_2-$ | $-CH_3$ | $n_D^{20}$ 1.5563 |
| 12 | 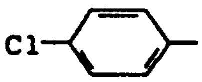 | $-S-CH_2-$ | $-CH_3$ | $n_D^{20}$ 1.5730 |
| 13 | 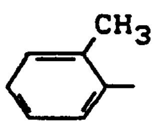 | -O- | $-CH_3$ | $^1$H-NMR*): |
| 14 | 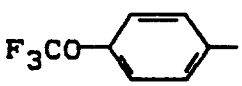 | $-CH_2-$ | 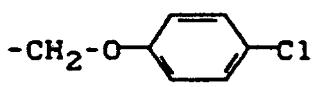 | Fp. 42-44° C |
| 15 | 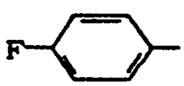 | $-CH_2-$ | $-CH_3$ | Fp. 126-128° C |

| Bsp. Nr. | Ar | $-(X)_m-$ | R | physikalische Eigenschaften |
|---|---|---|---|---|
| 16 | 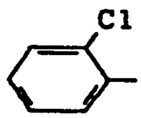 | $-CH_2-$ | $-CH_3$ | $n_D^{20}$ 1.5592 |
| 17 | 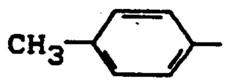 | $-CH_2-$ | $-CH_3$ | $n_D^{20}$ 1.5458 |
| 18 | 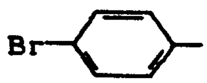 | $-CH_2-$ | $-CH_3$ | $n_D^{20}$ 1.5586 |
| 19 | 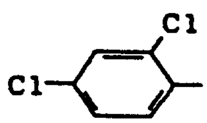 | $-CH_2-$ | $-CH_3$ | $n_D^{20}$ 1.5715 |
| 20 | 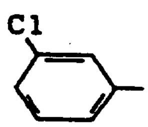 | $-CH_2-$ | $-CH_3$ | $n_D^{20}$ 1.5691 |
| 21 | 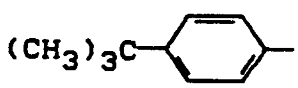 | -S- | $-CH_3$ | Fp. 121-123° C |
| 22 | 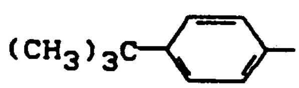 | -O- | $-CH_3$ | Fp. 145 °C |
| 23 | 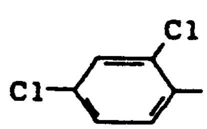 | -O- | $-CH_3$ | $^1$H-NMR*): 1.85(s); 1.37(s); 1.20(s) |

| Bsp. Nr. | Ar | $-(X)_m-$ | R | physikalische Eigenschaften |
|---|---|---|---|---|
| 24 | 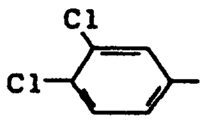 | -O- | $-CH_3$ | Fp. 172° C |
| 25 | 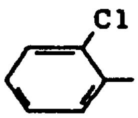 | -O- | $-CH_3$ | $^1H$-NMR*): 1,85(s); 1,40(s); 1,20(s) |
| 26 | 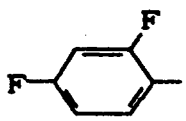 | -O- | $-CH_3$ | Fp. 134 °C |
| 27 | 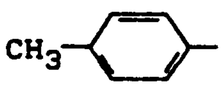 | -O- | $-CH_3$ | Fp. 155 °C |
| 28 | 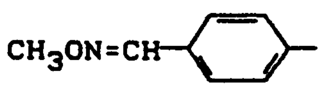 | -O- | $-CH_3$ | Fp. 122-123 °C |
| 29 | 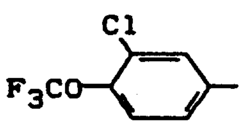 | $-CH_2-$ | $-CH_3$ | $^1H$-NMR*): 1.72(s); 0,80(s); 0,87(s) |
| 30 | 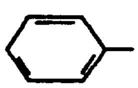 | -O- | $-CH_3$ | Fp. 102 °C |
| 31 | 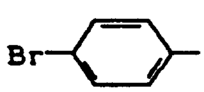 | -O- | $-CH_3$ | Fp. 141-143 °C |

| Bsp. Nr. | Ar | -(X)$_m$- | R | physikalische Eigenschaften |
|---|---|---|---|---|
| 32 | 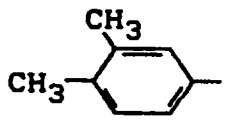 | -O- | $-CH_3$ | Fp. 151-152 °C |
| 33 | 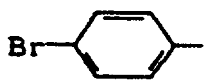 | -S- | $-CH_3$ | Fp. 127-128 °C |
| 34 | 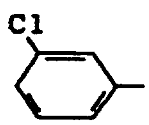 | -O- | $-CH_3$ | Fp. 108 °C |
| 35 | 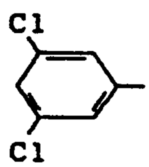 | -O- | $-CH_3$ | Fp. 138 °C |
| 36 | 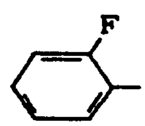 | -O- | $-CH_3$ | Fp. 91 °C |
| 37 | 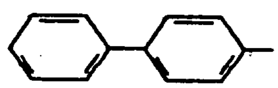 | -O- | $-CH_3$ | Fp. 164 °C |
| 38 | 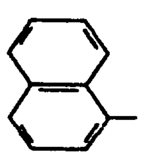 | -O- | $-CH_3$ | Fp. 121 °C |

| Bsp. Nr. | Ar | $-(X)_m-$ | R | physikalische Eigenschaften |
|---|---|---|---|---|
| 39 | 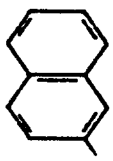 | -O- | $-CH_3$ | Fp. 159 °C |
| 40 | 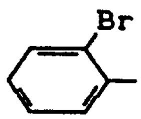 | -O- | $-CH_3$ | $n_D^{20}$ 1.5671 |
| 41 | 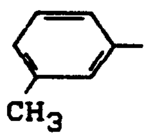 | -O- | $-CH_3$ | Fp. 67-69° C |
| 42 | 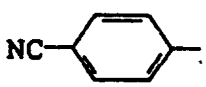 | $-O-CH_2$ | $-CH_3$ | $^1$H-NMR*): |
| 43 | 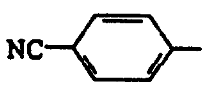 | $-S-CH_2$ | $-CH_3$ | $^1$H-NMR*): |
| 44 | 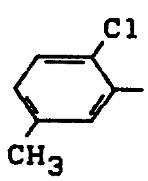 | -O- | $-CH_3$ | Fp. 145° C |
| 45 | 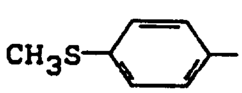 | -O- | $-CH_3$ | Fp. 124° C |
| 46 | 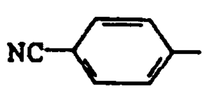 | -O- | $-CH_3$ | Fp. 156° C |

| Bsp. Nr. | Ar | -(X)$_m$- | R | physikalische Eigenschaften |
|---|---|---|---|---|
| 47 | 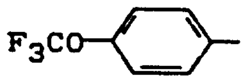 | -O- | $CH_3$ | Fp. 118° C |
| 48 | 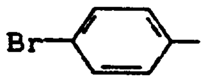 | $-O-CH_2-$ | $CH_3$ | Fp. 109-113° C |
| 49 | 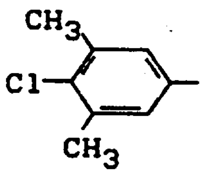 | -O- | $CH_3$ | Fp. 173° C |
| 50 | 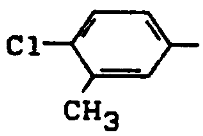 | -O- | $CH_3$ | Fp. 145° C |
| 51 | 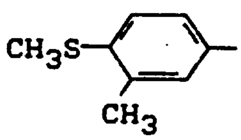 | -O- | $CH_3$ | Fp. 115° C |
| 52 | 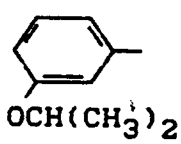 | -O- | $CH_3$ | Öl |
| 53 | 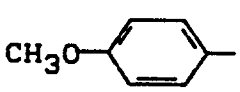 | -O- | $CH_3$ | Fp. 145° C |
| 54 | 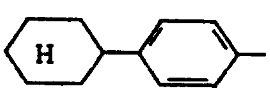 | -O- | $CH_3$ | Fp. 103° C |

| Bsp. Nr. | Ar | $-(X)_m-$ | R | physikalische Eigenschaften |
|---|---|---|---|---|
| 55 | 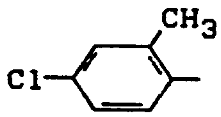 | -O- | $CH_3$ | $n_D^{20}$ 1.5491 |
| 56 | 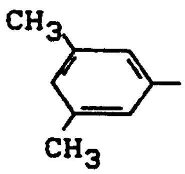 | -O- | $CH_3$ | Fp. 106° C |
| 57 | 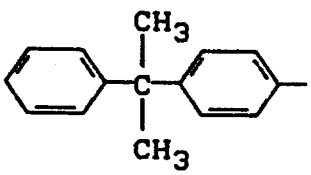 | -O- | $CH_3$ | $n_D^{20}$ 1.5723 |
| 58 | 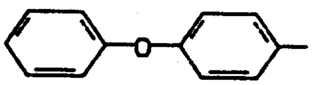 | -O- | $CH_3$ | Fp. 129-131° C |
| 59 | 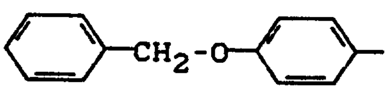 | -O- | $CH_3$ | Fp. 103-105° C |
| 60 | 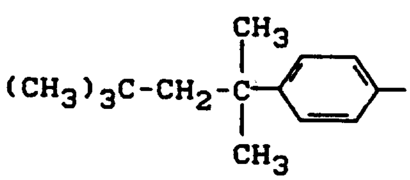 | -O- | $CH_3$ | Fp. 131-133° C |
| 61 | 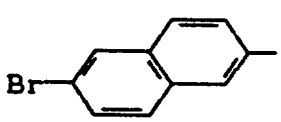 | -O- | $CH_3$ | Fp. 123° C |

| Bsp. Nr. | Ar | -(X)$_m$- | R | physikalische Eigenschaften |
|---|---|---|---|---|
| 62 | 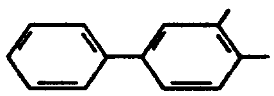 | -O- | $CH_3$ | $n_D^{20}$ 1.5961 |

*) Die $^1$H-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

Anwendungsbeispiele

In den folgenden Anwendungsbeispielen wurden die nachstehend aufgeführten Verbindungen als Vergleichsubstanzen eingesetzt:

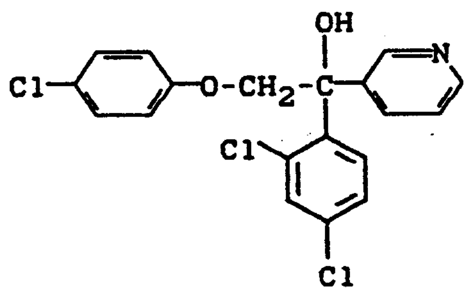 x $\frac{1}{2}$ 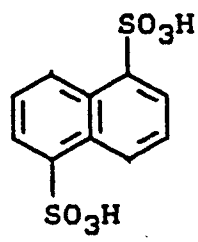 (A)

2-(4-Chlorphenoxy)-1-(2,4-dichlorphenyl)-1-(3-pyridyl)ethanol Naphthalindisulfonat

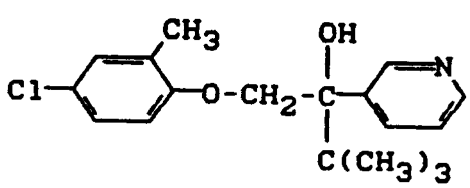 (B)

1-(4-Chlor-2-methylphenoxy)-3,3-dimethyl-2-(3-pyridyl)-2-butanol

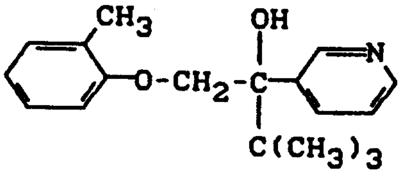

(C)

3,3-Dimethyl-1-(2-methylphenoxy)-2-(3-pyridyl)-2-butanol

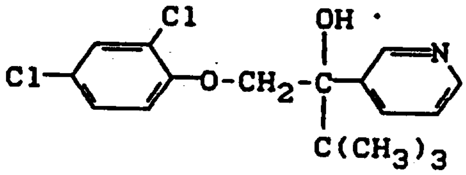

(D)

1-(2,4-Dichlorphenoxy)-3,3-dimethyl-2-(3-pyridyl)-2-butanol
(alle bekannt aus EP-A 0 001 399).

Beispiel A

Botrytis-Test (Bohne)/protektiv

| | |
|---|---|
| Lösungsmittel: | 4,7 Gewichtsteile Aceton |
| Emulgator: | 0,3 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20 °C aufgestellt. 3 Tage nach der Inokulation wird die Größe der Befallsflecken auf den Blättern ausgewertet.

Eine deutliche Überlegenheit in der Wirksamkeit gegenüber dem Stand der Technik zeigen in diesem Test z.B. die Verbindungen gemäß den Herstellungsbeispielen: 1, 3, 5, 7, 8 und 15.

Beispiel B

Erysiphe-Test (Gerste) / protektiv

| | |
|---|---|
| Lösungsmittel: | 100 Gewichtsteile Dimethylformamid |
| Emulgator: | 0,25 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20 °C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigt bei diesem Test z.B. die Verbindung gemäß Herstellungsbeispiel 3.

Beispiel C

Pyricularia-Test (Reis) /protektiv

| | |
|---|---|
| Lösungsmittel: | 12,5 Gewichtsteile Aceton |
| Emulgator: | 0,3 Gewichtsteile Alkylarylpolyglykolether |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25 °C aufgestellt.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß den Herstellungsbeispielen: 1, 3, 4, 10, 18 und 19.

Beispiel D

Pellicularia-Test (Reis)

| | |
|---|---|
| Lösungsmittel: | 12,5 Gewichtsteile Aceton |
| Emulgator: | 0,3 Gewichtsteile Alkylarylpolyglykolether |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf Wirksamkeit werden junge Reispflanzen im 3- bis 4-Blattstadium tropfnaß gespritzt. Die Pflanzen verbleiben bis zum Abtrocknen im Gewächshaus. Anschließend werden die Pflanzen mit Pellicularia sasakii inokuliert und bei 25 °C und 100 % relativer Luftfeuchtigkeit aufgestellt.

5 bis 8 Tage nach der Inokulation erfolgt die Auswertung des Krankheitsbefalles.

Eine deutliche Überlegenheit in der Wirksamkeit gegenüber dem Stand der Technik zeigen bei diesem Test z.B. die Verbindungen gemäß den Herstellungsbeispielen: 1 und 10.

Beispiel E

Pyrenophora teres-Test (Gerste) / protektiv

| | |
|---|---|
| Lösungsmittel: | 100 Gewichtsteile Dimethylformamid |
| Emulgator: | 0,25 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Abtrocknen des Spritzbelages werden die Pflanzen mit einer Konidiensuspension von Pyrenophora teres besprüht. Die Pflanzen verbleiben 48 Stunden bei 20 °C und 100 % relativer Luftfeuchtigkeit in einer Inkubationskabine.

Die Pflanzen werden in einem Gewächshaus bei einer Temperatur von ca. 20 °C und einer relativen Luftfeuchtigkeit von ca. 80 % aufgestellt.

7 Tage nach der Inokulation erfolgt die Auswertung.

Die Verbindungen zeigen gegenüber dem Stand der Technik eine hohe Überlegenheit, so die präparativen Beispiele: 3, 4, 5, 8, 15, 25, 26, 29, 28, 47, 49, 50, 51 und 53.

Beispiel F

Cochliobolus sativus-Test (Gerste) / protektiv

| | |
|---|---|
| Lösungsmittel: | 100 Gewichtsteile Dimethylformamid |
| Emulgator: | 0,25 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Cochliobolus sativus besprüht. Die Pflanzen verbleiben 48 Stunden bei 20 °C und 100 °C rel. Luftfeuchtigkeit in einer Inkubationskabine.

Die Pflanzen werden in einem Gewächshaus bei einer Temperatur von 20 °C und einer relativen Luftfeuchtigkeit von ca. 80 % aufgestellt.

7 Tage nach der Inokulation erfolgt die Auswertung.

Eine deutliche Überlegenheit in der Wirksamkeit gegenüber den Stand der Technik zeigen bei diesem Test z.B. die Verbindungen gemäß den Herstellungsbeispielen: 3, 4, 5, 1, 15, 17, 31, 47, 39, 41, 49, 50, 51, 52 und 53.

Beispiel G

Leptosphaeria nodorum-Test (Weizen) / protektiv

| | |
|---|---|
| Lösungsmittel: | 100 Gewichtsteile Dimethylformamid |
| Emulgator: | 0,25 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Leptosphaeria nodorum besprüht. Die Pflanzen verbleiben 48 Stunden bei 20 °C und 100 % relativer Luftfeuchtigkeit in einer Inkubationskabine.

Die Pflanzen werden in einem Gewächshaus bei einer Temperatur von ca. 15 °C und einer relativen Luftfeuchtigkeit von ca. 80 % aufgestellt.

10 Tage nach der Inokulation erfolgt die Auswertung.

Eine deutliche Überlegenheit in der Wirksamkeit gegenüber dem Stand der Technik zeigen bei diesem Test z.B. die Verbindungen gemäß den Herstellungsbeispielen: 3, 4, 5, 1, 8, 17, 20, 26, 29, 47, 39 und 48.

Beispiel H

Puccinia-Test (Weizen) / protektiv

| | |
|---|---|
| Lösungsmittel: | 100 Gewichtsteile Dimethylformamid |
| Emulgator: | 0,25 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit einer Sporensuspension von Puccinia recondita mit in einer 0,1 %igen wäßrigen Agarlösung inokuliert. Nach Antrocknen besprüht man die Pflanzen mit der Wirkstoffzubereitung taufeucht. Die Pflanzen verbleiben 24 Stunden bei 20 °C und 100 % rel. Luftfeuchtigkeit in einer Inkubationskabine.

Die Pflanzen werden in einem Gewächshaus bei einer Temperatur von ca. 20 °C und einer relativen Luftfeuchtigkeit von ca. 80 % aufgestellt, um die Entwicklung von Rostpusteln zu begünstigen.

10 Tage nach der Inkubation erfolgt die Auswertung.

Eine deutliche Überlegenheit in der Wirksamkeit gegenüber dem Stand der Technik zeigen bei diesem Test z.B. die Verbindungen gemäß den Herstellungsbeispielen: 9, 10, 11, 2, 14, 26, 47 und 56.

**Patentansprüche**

**1.** Substituierte Pyridin-Derivate der allgemeinen Formel (I),

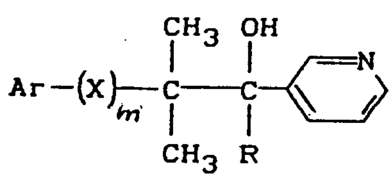

(I)

in welcher

Ar für gegebenenfalls einfach bis mehrfach, gleich oder verschieden substituiertes Phenyl steht, wobei als Substituenten infrage kommen: Halogen, Cyano, Nitro, jeweils geradkettiges oder verzweigtes Alkyl, Alkoxy, Alkylthio, Alkylsulfinyl oder Alkylsulfonyl mit jeweils 1 bis 8 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen, Cycloalkyl mit 3 bis 6 Kohlensloffatomen, gegebenenfalls einfach bis mehrfach, gleich oder verschieden durch Halogen substituiertes, zweifach verknüpftes Dioxyalkylen oder jeweils gegebenenfalls einfach bis mehrfach, gleich oder verschieden im Phenylteil durch Halogen substituiertes Phenyl, Phenoxy, Phenylalkyl oder Phenyllkoxy mit jeweils gegebenenfalls 1 bis 4 Kohlenstoffatomen im geradkettigen oder verzweigten Alkyl- oder Alkoxyteil; oder für gegebenenfalls einfach bis mehrfach, gleich oder verschieden substituiertes Naphthyl steht, wobei als Substituenten infrage kommen: Halogen, Cyano oder geradkettiges oder verzweigtes Alkyl mit 1 bis 4 Kohlenstoffatomen;

X für Sauerstoff, Schwefel, Sulfinyl, Sulfonyl oder für eine der Gruppen $-CH_2-$; $-O-CH_2-$; $-CH_2-O-$; $-O-CH_2-CH_2-$; $-S(O)_n-CH_2-$; $-CH_2-S(O)_n-$oder $-S(O)_n-CH_2CH_2-$ steht,
wobei

n jeweils für eine Zahl 0, 1 oder 2 steht,

R für jeweils geradkettiges oder verzweigtes Alkyl mit 1 bis 6 Kohlenstoffatomen, oder für jeweils gegebenenfalls einfach bis mehrfach, gleich oder verschieden substituiertes Phenoxymethyl oder Phenylthiomethyl steht, wobei als Phenylsubstituenten jeweils die bei dem Rest Ar genannten infrage kommen und

m für eine Zahl 0 oder 1 steht,
sowie deren pflanzenverträgliche Säureadditionssalze, Metallsalzkomplexe,

2. Substituierte Pyridin-Derivate gemäß Anspruch 1, wobei in der Formel (I)

Ar für gegebenenfalls ein- bis dreifach, gleich oder verschieden substituiertes Phenyl steht, wobei als Substituenten infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Ethylthio, Trifluormethyl, Dichlorfluormethyl, Difluorchlormethyl, Difluorbrommethyl, Trichlormethyl, Fluormethyl, Difluormethyl, Trifluorethyl, Tetrafluorethyl, Trifluorchlorethyl, Pentafluorethyl, Trifluormethoxy, Difluormethoxy, Fluormethoxy, Dichlorfluormethoxy, Difluorchlormethoxy, Difluorbrommethoxy, Trichlormethoxy, Trifluorethoxy, Tetrafluorethoxy, Pentafluorethoxy, Trifluorchlorethoxy, Trifluordichlorethoxy, Difluortrichlorethoxy, Pentachlorethoxy, Trifluormethylthio, Difluormethylthio, Fluormethylthio, Difluorchlormethylthio, Dichlorfluormethylthio, Difluorethylthio, Difluorbrommethylthio, Trichlormethylthio, Trifluorethylthio, Tetrafluorethylthio, Pentafluorethylthio, Trifluorchlorethylthio, Trifluordichlorethylthio, Pentachlorethylthio, Methylsulfinyl, Trifluormethylsulfinyl, Dichlorfluormethylsulfinyl, Difluorchlormethylsulfinyl, Fluormethylsulfinyl, Difluormethylsulfinyl, Methylsulfonyl, Trifluormethylsulfonyl, Dichlorfluormethylsulfonyl, Difluorchlormethylsulfonyl, Fluormethylsulfonyl, Difluormethylsulfonyl, Methoxycarbonyl, Ethoxycarbonyl, Methoximinomethyl, Ethoximinomethyl, Methoximinoethyl, Ethoximinoethyl, Difluordioxymethylen, Tetrafluordioxyethylen, Trifluordioxyethylen, Difluordioxyethylen, Dioxymethylen, Dioxyethylen sowie jeweils gegebenenfalls ein- bis dreifach durch Fluor oder Chlor substituiertes Phenyl oder Phenoxy;
oder für gegebenenfalls ein- bis dreifach, gleich oder verschieden substituiertes $\alpha$-Naphthyl oder $\beta$-Naphthyl steht, wobei als Substituenten jeweils infrage kommen: Fluor, Chlor, Brom, Cyano, Methyl, Ethyl, n- oder i-Propyl;

X für Sauerstoff, Schwefel, Sulfinyl, Sulfonyl oder für eine der Gruppen $-CH_2-$; $-O-CH_2-$; $-CH_2-O-$; $-O-CH_2-CH_2-$; $-S(O)_n-CH_2-$; $-CH_2-S(O)_n-$ oder $-S(O)_n-CH_2-CH_2-$ steht, wobei

n jeweils für eine Zahl 0, 1 oder 2 steht,

R für Methyl, Ethyl, n- oder i-Propyl, n-, i-, s- oder t-Butyl oder für jeweils gegebenenfalls ein- bis dreifach, gleich oder verschieden substituiertes Phenoxymethyl oder Phenylthiomethyl steht, wobei als Phenylsubstituenten jeweils die bei dem Rest Ar genannten infrage kommen und

m für eine Zahl 0 oder 1 steht.

3. Verfahren zur Herstellung von substituierten Pyridin-Derivaten der allgemeinen Formel (I) gemäß Anspruch 1
sowie von deren pflanzenverträglichen Säureadditions-Salzen und Metallsalz-komplexen, dadurch gekennzeichnet, daß man

(a) Ketone der Formel (II),

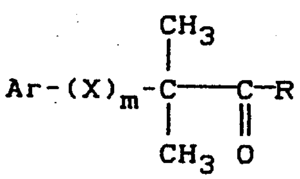

(II)

in welcher

Ar, X, R und m die oben angegebene Bedeutung haben,

mit Pyridyl-Grignard-Verbindungen der Formel (III),

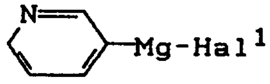

(III)

in welcher
Hal[1] für Chlor, Brom oder Iod steht,
gegebenenfalls in Gegenwart eines Verdünnungsmittels umsetzt oder daß man
(b) Pyridylketone der Formel (IV),

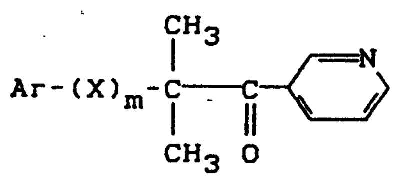
(IV)

in welcher
Ar, X und m die oben angegebene Bedeutung haben,
mit Alkyl-Grignard-Verbindungen der Formel (V),

R-Mg-Hal[2] (V)

in welcher
R die oben angegebene Bedeutung hat und
Hal[2] für Chlor, Brom oder Iod steht,
gegebenenfalls in Gegenwart eines Verdünnungsmittels umsetzt oder daß man alternativ auch, um substituierte Pyridin-Derivate der Formel (Ia),

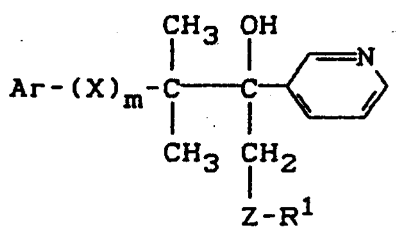
(Ia)

in welcher
Z für Sauerstoff oder Schwefel steht,
R[1] für gegebenenfalls einfach bis mehrfach, gleich oder verschieden substituiertes Phenyl steht, wobei als Substituenten die beim Rest Argenannten infrage kommen und
Ar, X und m die oben angegebenen Bedeutungen haben,
zu erhalten,
(c) Pyridyloxirane der Formel (VI),

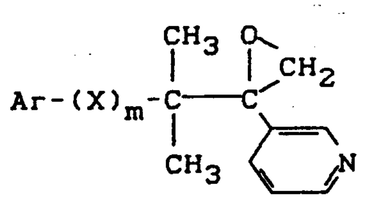
(VI)

in welcher
Ar, X und m die oben angegebene Bedeutung haben,
mit Alkoholen oder Thiolen der Formel (VII),

R[1]-ZH (VII)

in welcher

$R^1$ und Z die oben angegebene Bedeutung haben,

gegebenenfalls in Gegenwart eines Verdünnungsmittels sowie gegebenenfalls in Gegenwart eines Reaktionshilfsmittel umsetzt und gegebenenfalls anschließend eine Säure oder ein Metallsalz addiert.

**4.** Schädlingsbekämpfungsmittel, gekennzeichnet durch einen Gehalt an mindestens einem substituierten Pyridin-Derivat der Formel (I) nach Anspruch 1 .

**5.** Verwendung von substituierten Pyridin-Derivaten der Formel (I) nach Anspruch 1 zur Bekämpfung von Schädlingen.

**6.** Verfahren zur Bekämpfung von Schädlingen, dadurch gekennzeichnet, daß man substituierte Pyridin-Derivate der Formel (I) nach Anspruch 1 auf Schädlinge und/oder ihren Lebensraum einwirken läßt.

**7.** Verfahren zur Herstellung von Schädlingsbekämpfungsmitteln, dadurch gekennzeichnet, daß man substituierte Pyridin-Derivate der Formel (I) nach Anspruch 1 mit Streckmitteln und/oder oberflächenaktiven Mitteln vermischt.

## Claims

**1.** Substituted pyridine derivatives of the general formula (I)

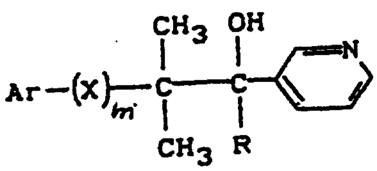

(I)

in which

Ar represents phenyl which is optionally monosubstituted to polysubstituted by identical or different substituents, suitable substituents being:
halogen, cyano, nitro, in each case straight-chain or branched alkyl, alkoxy, alkylthio, alkylsulphinyl or alkylsulphonyl, each having 1 to 8 carbon atoms, in each case straight-chain or branched halogenoalkyl, halogenoalkoxy, halogenoalkylthio, halogenoalkylsulphinyl or halogenoalkylsulphonyl, each having 1 to 4 carbon atoms and 1 to 9 identical or different halogen atoms, in each case straight-chain or branched alkoxycarbonyl or alkoximinoalkyl, each having 1 to 4 carbon atoms in the individual alkyl parts, cycloalkyl having 3 to 6 carbon atoms, divalent dioxyalkylene which is optionally monosubstituted to polysubstituted by identical or different halogen substituents, or phenyl, phenoxy, phenylalkyl or phenylalkoxy, each of which is optionally monosubstituted to polysubstituted in the phenyl part by identical or different halogen substituents and each of which optionally has 1 to 4 carbon atoms in the straight-chain or branched alkyl or alkoxy part; or represents naphthyl which is optionally monosubstituted to polysubstituted by identical or different substituents, suitable substituents being: halogen, cyano or straight-chain or branched alkyl having 1 to 4 carbon atoms;

X represents oxygen, sulphur, sulphinyl, sulphonyl or one of the groups $-CH_2-$; $-O-CH_2-$; $-CH_2-O-$; $-O-CH_2-CH_2-$; $-S(O)_n-CH_2-$; $-CH_2-S(O)_n-$ or $-S(O)_n-CH_2-CH_2-$,
in which

n in each case represents the number 0, 1 or 2,

R in each case represents straight-chain or branched alkyl having 1 to 6 carbon atoms or represents phenoxymethyl or phenylthiomethyl, each of which is optionally monosubstituted to polysubstituted by identical or different substituents, suitable phenyl substituents in each case being those mentioned for the radical Ar and

m represents the number 0 or 1,

and their plant-compatible acid addition salts and metal salt complexes.

2. Substituted pyridine derivatives according to Claim 1, in which in the formula (I)

Ar represents phenyl which is optionally monosubstituted, disubstituted or trisubstituted by identical or different substituents, suitable substituents being: fluorine, chlorine, bromine, cyano, nitro, methyl, ethyl, n- or i-propyl, n-, i-, s- or t-butyl, methoxy, ethoxy, n- or i-propoxy, methylthio, ethylthio, trifluoromethyl, dichlorofluoromethyl, difluorochloromethyl, difluorobromomethyl, trichloromethyl, fluoromethyl, difluoromethyl, trifluoroethyl, tetrafluoroethyl, trifluorochloroethyl, pentafluoroethyl, trifluoromethoxy, difluoromethoxy, fluoromethoxy, dichlorofluoromethoxy, difluorochloromethoxy, difluorobromomethoxy, trichloromethoxy, trifluoroethoxy, tetrafluoroethoxy, pentafluoroethoxy, trifluorochloroethoxy, trifluorodichloroethoxy, difluorotrichloroethoxy, pentachloroethoxy, trifluoromethylthio, difluoromethylthio, fluoromethylthio, difluorochloromethylthio, dichlorofluoromethylthio, difluoroethylthio, difluorobromomethylthio, trichloromethylthio, trifluoroethylthio, tetrafluoroethylthio, pentafluoroethylthio, trifluorochloroethylthio, trifluorodichloroethylthio, pentachloroethylthio, methylsulphinyl, trifluoromethylsulphinyl, dichlorofluoromethylsulphinyl, difluorochloromethylsulphinyl, fluoromethylsulphinyl, difluoromethylsulphinyl, methylsulphonyl, trifluoromethylsulphonyl, dichlorofluoromethylsulphonyl, difluorochloromethylsulphonyl, fluoromethylsulphonyl, difluoromethylsulphonyl, methoxycarbonyl, ethoxycarbonyl, methoximinomethyl, ethoximinomethyl, methoximinoethyl, ethoximinoethyl, difluorodioxymethylene, tetrafluorodioxyethylene, trifluorodioxyethylene, difluorodioxyethylene, dioxymethylene, dioxyethylene and also phenyl or phenoxy, each of which is optionally monosubstituted, disubstituted or trisubstituted by fluorine or chlorine; or represents $\alpha$-naphthyl or $\beta$-naphthyl, each of which is optionally monosubstituted, disubstituted or trisubstituted by identical or different substituents, suitable substituents in each case being: fluorine, chlorine, bromine, cyano, methyl, ethyl, n- or i-propyl;

X represents oxygen, sulphur, sulphinyl, sulphonyl, or one of the groups $-CH_2-$; $-O-CH_2-$; $-CH_2-O-$; $-O-CH_2-CH_2-$; $-S(O)_n-CH_2$; $-CH_2-S(O)_n-$ or $-S(O)_n-CH_2-CH_2-$,
in which

n in each case represents the number 0, 1 or 2,

R represents methyl, ethyl, n- or i-propyl, n-, i-, s- or t-butyl or represents phenoxymethyl or phenylthiomethyl, each of which is optionally monosubstituted, disubstituted or trisubstituted by identical or different substituents, suitable phenyl substituents in each case being those mentioned for the radical Ar, and

m represents the number 0 to 1.

3. Process for the preparation of substituted pyridine derivatives of the general formula (I) according to Claim 1 and of their plant-compatible acid addition salts and metal salt complexes, characterized in that
(a) ketones of the formula (II)

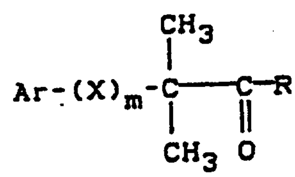

(II)

in which
Ar, X , R and m have the abovementioned meaning,
are reacted with pyridyl Grignard compounds of the formula (III)

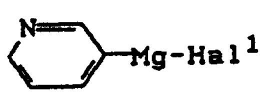

(III)

in which
Hal 1 represents chlorine, bromine or iodine,

if appropriate in the presence of a diluent or in that
(b) pyridyl ketones of the formula (IV)

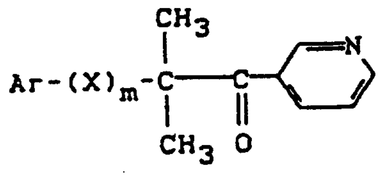
(IV)

in which
Ar, X and m have the abovementioned meaning,
are reacted with alkyl Grignard compounds of the formula (V),

R-Mg-$Hal^2$ (V)

in which
R has the abovementioned meaning and
$Hal^2$ represents chlorine, bromine or iodine,
if appropriate in the presence of a diluent or also alternatively, in that, in order to obtain substituted pyridine derivatives of the formula Ia

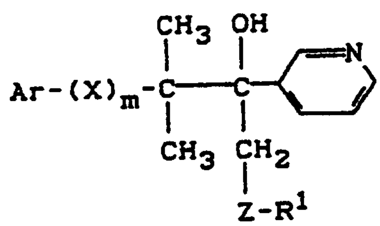
(Ia)

in which
Z represents oxygen or sulphur,
$R^1$ represents phenyl which is optionally monosubstituted to polysubstituted by identical or different substituents, suitable substituents being those mentioned for the radical Ar, and
Ar, X and m have the abovementioned meanings,
(c) pyridyl oxiranes of the formula (VI)

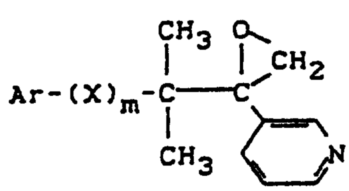
(VI)

in which
Ar, X and m have the abovementioned meaning,
are reacted with alcohols or thiols of the formula (VII)

$R^1$-ZH (VII)

in which
$R^1$ and Z have the abovementioned meaning,
if appropriate in the presence of a diluent and also if appropriate in the presence of a reaction

auxiliary and if appropriate an acid or a metal salt is then adducted.

**4.** Pesticides, characterized in that they contain at least one substituted pyridine derivative of the formula (I) according to Claim 1.

**5.** Use of substituted pyridine derivatives of the formula (I) according to Claim 1 for combating pests.

**6.** Method for combating pests, characterized in that substituted pyridine derivatives of the formula (I) according to Claim 1 are allowed to act on pests and/or their environment.

**7.** Process for the production of pesticides, characterized in that substitued pyridine derivatives of the formula (I) according to Claim 1 are mixed with extenders and/or surface-active agents.

**Revendications**

**1.** Dérivés substitués de pyridine de formule générale (I)

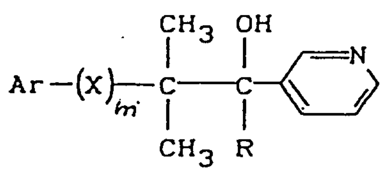

(I)

dans laquelle

Ar représente un groupe phényle portant le cas échéant un ou plusieurs substituants identiques ou différents, en considérant comme substituants : un halogène, un groupe cyano, nitro, un groupe alkyle, alkoxy, alkylthio, alkylsulfinyle ou alkylsulfonyle linéaire ou ramifié ayant chacun 1 à 8 atomes de carbone, un groupe halogénalkyle, halogénalkoxy, halogénalkylthio, halogénalkylsulfinyle ou halogénalkylsulfonyle linéaire ou ramifié ayant chacun 1 à 4 atomes de carbone et 1 à 9 atomes d'halogènes identiques ou différents, un groupe alkoxycarbonyle ou alkoximinoalkyle linéaire ou ramifié ayant chacun 1 à 4 atomes de carbone dans les parties alkyle individuelles, un groupe cycloalkyle de 3 à 6 atomes de carbone, un groupe dioxyalkylène divalent portant le cas échéant un ou plusieurs substituants halogéno identiques ou différents, ou un groupe phényle, phénoxy, phénylalkyle ou phénylalkoxy portant chacun le cas échéant un ou plusieurs substituants halogéno identiques ou différents dans la partie phényle et ayant chacun le cas échéant 1 à 4 atomes de carbone dans la partie alkyle ou alkoxy linéaire ou ramifiée ; ou un groupe naphtyle portant le cas échéant un ou plusieurs substituants identiques ou différents, en considérant comme substituants : un halogène, un radical cyano ou un radical alkyle linéaire ou ramifié ayant 1 à 4 atomes de carbone ;

X représente de l'oxygène, du soufre, un groupe sulfinyle, sulfonyle ou l'un des groupes $-CH_2-$ ; $-O-CH_2-$ ; $-CH_2-O-$ ; $-O-CH_2-CH_2-$ ; $-S(O)_n-CH_2-$ ; $-CH_2-S(O)_n-$ ou $-S(O)_n-CH_2CH_2-$, où

n a dans chaque cas la valeur 0, 1 ou 2,

R désigne un groupe alkyle linéaire ou ramifié de 1 à 6 atomes de carbone ou un groupe phénoxyméthyle ou phénylthiométhyle portant le cas échéant un ou plusieurs substituants identiques ou différents, en considérant comme substituants de chaque groupe phényle les substituants mentionnés pour le reste Ar et

m est le nombre 0 ou 1,

ainsi que leurs sels d'addition d'acides et leurs complexes de sels métalliques compatibles avec les plantes.

**2.** Dérivés substitués de pyridine suivant la revendication 1, dans la formule (I) desquels

Ar désigne un groupe phényle portant le cas échéant un à trois substituants identiques ou différents, en considérant comme substituants : le fluor, le chlore, le brome, un radical cyano, nitro, méthyle, éthyle, n-propyle, isopropyle, n-butyle, isobutyle, sec-butyle, tertiobutyle, méthoxy, éthoxy, n-propoxy, isopropoxy, méthylthio, éthylthio, trifluorométhyle, dichlorofluorométhyle, difluorochlorométhyle, difluorobromométhyle, trichlorométhyle, fluorométhyle, difluorométhyle, trifluoréthyle, tétrafluoréthyle, trifluorochloréthyle, pentafluoréthyle, trifluorométhoxy, difluorométhoxy, fluorométhoxy, dichlorofluorométhoxy, difluorochlorométhoxy, difluorobromométhoxy, trichlorméthoxy, trifluoréthoxy, tétrafluoréthoxy, pentafluoréthoxy, trifluorochloréthoxy, trifluorodichloréthoxy, difluorotrichloréthoxy, pentachloréthoxy, trifluorométhylthio, difluorométhylthio, fluorométhylthio, difluorochlorométhylthio, dichlorofluorométhylthio, difluoréthylthio, difluorobromométhylthio, trichlorométhylthio, trifluoréthylthio, tétrafluoréthylthio, pentafluoréthylthio, trifluorochloréthylthio, trifluorodichloréthylthio, pentachloréthylthio, méthylsulfinyle, trifluorométhylsulfinyle, dichlorofluorométhylsulfinyle, difluorochlorométhylsulfinyle, fluorométhylsulfinyle, difluorométhylsulfinyle, méthylsulfonyle, trifluorométhylsulfonyle, dichlorofluorométhylsulfonyle, difluorochlorométhylsulfonyle, fluorométhylsulfonyle, difluorométhylsulfonyle, méthoxycarbonyle, éthoxycarbonyle, méthoximinométhyle, éthoximinométhyle, méthoximinoéthyle, éthoximinoéthyle, difluorodioxyméthylène, tétrafluorodioxyéthylène, trifluorodioxyéthylène, difluorodioxyéthylène, dioxyméthylène, dioxyéthylène ainsi qu'un groupe phényle ou phénoxy portant chacun le cas échéant un à trois substituants fluoro ou chloro ;
ou un groupe α-naphtyle ou β-naphtyle portant le cas échéant un à trois substituants identiques ou différents, en considérant dans chaque cas comme substituants : le fluor, le chlore, le brome, un radical cyano, méthyle, éthyle, n-propyle ou isopropyle,

X représente l'oxygène, le soufre, un groupe sulfinyle, sulfonyle ou l'un des groupes $-CH_2-$ ; $-O-CH_2-$ ; $-CH_2-O-$ ; $-O-CH_2-CH_2-$ ; $-S(O)_n-CH_2-$ ; $-CH_2-S(O)_n-$ ou $-S(O)_n-CH_2CH_2-$, où

n représente dans chaque cas le nombre 0, 1 ou 2,

R est un groupe méthyle, éthyle, n-propyle, isopropyle, n-butyle, isobutyle, sec-butyle, tertiobutyle ou un groupe phénoxyméthyle ou phénylthiométhyle portant chacun le cas échéant un à trois substituants identiques ou différents, en considérant dans chaque cas comme substituants du groupe phényle les substituants mentionnés pour le reste Ar et

m représente le nombre 0 ou 1.

**3.** Procédé de production de dérivés substitués de pyridine de formule générale (I) suivant la revendication 1 ainsi que de leurs sels d'addition d'acides et de leurs complexes de sels métalliques compatibles avec les plantes, caractérisé en ce que :

(a) on fait réagir des cétones de formule (II),

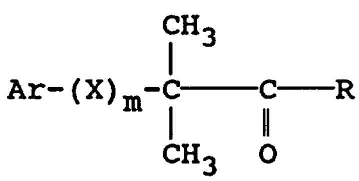

(II)

dans laquelle

Ar, X, R et m ont la définition indiquée ci-dessus,

avec des composés pyridyliques de Grignard de formule (III)

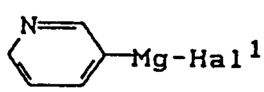

(III)

dans laquelle

$Hal^1$ représente du chlor, du brome ou de l'iode,

le cas échéant en présence d'un diluant, ou bien

(b) on fait réagir des pyridylcétones de formule (IV)

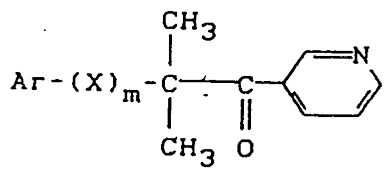

(IV)

dans laquelle

Ar, X et m ont la définition indiquée ci-dessus,

avec des composés alkyliques de Grignard de formule (V)

R-Mg-$Hal^2$ (V)

dans laquelle

R a la définition indiquée ci-dessus et

$Hal^2$ représente le chlore, le brome ou l'iode,

le cas échéant en présence d'un diluant, ou bien en variante, pour obtenir des dérivés substitués de pyridine de formule (Ia),

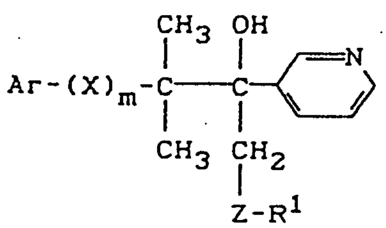

(Ia)

dans laquelle

Z est de l'oxygène ou du soufre,

$R^1$ est un groupe phényle portant le cas échéant un ou plusieurs substituants identiques ou différents, en considérant comme substituants ceux qui ont été mentionnés pour le reste Ar et

Ar, X et m ont la définition indiquée ci-dessus,

(c) on fait réagir des pyridyloxiranes de formule (VI)

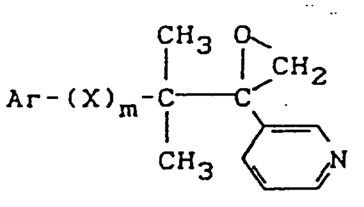

(VI)

dans laquelle

Ar, X et m ont la définition indiquée ci-dessus,

avec des alcools ou des thiols de formule (VII)

$R^1$-ZH (VII)

dans laquelle

$R^1$ et Z ont la définition indiquée ci-dessus,

le cas échéant en présence d'un diluant de même que, éventuellement, en présence d'une

substance auxiliaire de réaction et on additionne ensuite le cas échéant un acide ou un sel métallique.

**4.** Composition pesticide, caractérisée par une teneur en au moins un dérivé substitué de pyridine de formule (I) suivant la revendication 1.

**5.** Utilisation de dérivés substitués de pyridine de formule (I) suivant la revendication 1 pour combattre des parasites.

**6.** Procédé pour combattre des parasites, caractérisé en ce qu'on fait agir des dérivés substitués de pyridine de formule (I) suivant la revendication 1 sur les parasites et/ou sur leur milieu.

**7.** Procédé de préparation de compositions pesticides, caractérisé en ce qu'on mélange des dérivés substitués de pyridine de formule (I) suivant la revendication 1 avec des diluants et/ou des agents tensio-actifs.